(19) Europäisches Patentamt
European Patent Office
Office européen des brevets

(11) EP 0 958 052 B1

(12) EUROPÄISCHE PATENTSCHRIFT

(45) Veröffentlichungstag und Bekanntmachung des Hinweises auf die Patenterteilung:
**04.12.2002 Patentblatt 2002/49**

(21) Anmeldenummer: **98903021.8**

(22) Anmeldetag: **22.01.1998**

(51) Int Cl.[7]: **B01J 31/02**, C07C 17/20

(86) Internationale Anmeldenummer:
**PCT/EP98/00332**

(87) Internationale Veröffentlichungsnummer:
**WO 98/032532 (30.07.1998 Gazette 1998/30)**

# (54) AMIDOPHOSPHONIUMSALZ ENTHALTENDER KATALYSATOR FÜR HALEX-REAKTIONEN

AMIDOPHOSPHONIUM-SALT-CONTAINING CATALYST FOR HALOGEN-FLUORINE EXCHANGE REACTIONS

CATALYSEUR CONTENANT UN SEL D'AMIDOPHOSPHONIUM POUR DES REACTIONS D'ECHANGE D'HALOGENE PAR DU FLUOR

(84) Benannte Vertragsstaaten:
**BE CH DE ES FR GB IE IT LI NL**

(30) Priorität: **23.01.1997 DE 19702282**

(43) Veröffentlichungstag der Anmeldung:
**24.11.1999 Patentblatt 1999/47**

(73) Patentinhaber: **Clariant GmbH**
**65929 Frankfurt am Main (DE)**

(72) Erfinder:
- **PASENOK, Sergej**
  **D-65779 Kelkheim (DE)**
- **APPEL, Wolfgang**
  **D-65779 Kelkheim (DE)**
- **PFIRMANN, Ralf**
  **D-64347 Griesheim (DE)**
- **WESSEL, Thomas**
  **D-60386 Frankfurt (DE)**
- **SCHACH, Thomas**
  **TARDEO MUMBAI 400034 (IN)**
- **SCHUBERT, Hans**
  **D-65779 Kelkheim (DE)**

(74) Vertreter: **Brundin, Eike**
**Clariant Service GmbH**
**Patente, Marken, Lizenzen**
**Am Unisys-Park 1**
**65843 Sulzbach (DE)**

(56) Entgegenhaltungen:
**EP-A- 0 523 671**
**WO-A-98/05610**
**US-A- 5 502 235**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 958 052 B1

## Beschreibung

Die vorliegende Erfindung betrifft ein gegenüber dem Stand der Technik verbessertes Katalysatorsystem zur Herstellung von Fluor enthaltenden Verbindungen mittels einer Halogen-Fluor-Austauschreaktion.

Fluor enthaltende Verbindungen finden unter anderem in flüssigkristallinen Mischungen Anwendung (EP 0 602 596).

Die Halogen-Fluor-Austauschreaktion ist auch unter dem Namen Halex-Reaktion bekannt. Sie stellt eine häufig praktizierte Methode dar, Fluor-Substituenten in eine Verbindung, die gegen Fluor austauschbares Halogen enthält, einzuführen.

Bei aromatischen Verbindungen, insbesondere bei aktivierten aromatischen Verbindungen, verläuft der Halogen-Fluor-Austausch im Sinne einer nucleophilen Substitution. Zur Durchführung dieser Reaktion werden vergleichweise hohe Reaktionstemperaturen benötigt, die häufig zwischen 200 und 300°C liegen, wodurch zum Teil erhebliche Anteile an Zersetzungsprodukten entstehen. Im allgemeinen kann auf ein Lösungsmittel nicht verzichtet werden, so daß die Raum/Zeitausbeuten im Vergleich zu lösungsmittelfreien Verfahren deutlich niedriger liegen. Als Alternative hierzu können herkömmliche Phasentransfer-Katalysatoren verwendet werden, durch die sich einige der vorstehend genannten Nachteile verringern lassen.

Andere Probleme, wie eine schlechte Rührbarkeit der Reaktionssuspension bei lösungsmittelfreien Verfahren, bleiben weiter bestehen. Bislang wurden als Phasentransfer-Katalysatoren quartäre Alkylammonium- oder Alkylphosphoniumsalze (US-PS 4287374), Pyridiniumsalze (WO 87/04194), Kronenether oder Tetraphenylphosphoniumsalze (J.H. Clark et al., Tetrahedron Letters 28 [1987], Seiten 111 bis 114) verwendet. Diese Phasentransferkatalysatoren weisen zum Teil vergleichsweise geringe Aktivität auf und sind unter den für die Durchführung der Reaktion erforderlichen Temperaturen nur mäßig stabil.

In Anbetracht dieser Einschränkungen und Nachteile besteht ein großes Bedürfnis nach einem verbesserten Katalysatorsystem, durch das die den bekannten Verfahren innewohnenden Nachteile, insbesondere hohe Reaktionstemperaturen und lange Reaktionszeiten, vermieden und zudem die gewünschten Fluor enthaltenden Verbindungen, insbesondere auch nicht-aktivierte aromatische Verbindungen, in guter bis sehr guter Ausbeute bei niedrigeren Reaktionstemperaturen und kürzeren Reaktionszeiten erhalten werden.

Es wurde gefunden, daß ein Gemisch aus einem Amidophosphoniumsalz der Formel (I) mit einem oder mehreren Verbindungen aus der Gruppe der quartären Ammoniumsalze, quartären Phosphoniumsalze und/oder der Polyether überraschenderweise die vorstehend genannten Forderungen erfüllt.

Gegenstand der vorliegenden Erfindung ist ein Katalysator für Halogen-Fluor-Austauschreaktionen an Aromaten, bestehend im wesentlichen aus einer Mischung aus einer oder mehreren Verbindungen der Komponente a) sowie mindestens einer Verbindung der Komponenten b), c) und/oder d), wobei die Komponenten

a) ein Amidophosphoniumsalz der Formel (I)

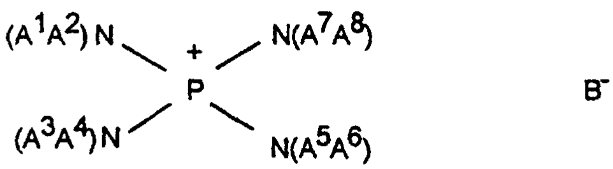

(I)

worin $A^1$, $A^2$, $A^3$, $A^4$, $A^5$, $A^6$, $A^7$, $A^8$ unabhängig voneinander, gleich oder verschieden sind und für ein geradkettiges oder verzweigtes Alkyl oder Alkenyl mit 1 bis 12 Kohlenstoffatomen, Cycloalkyl mit 4 bis 8 Kohlenstoffatomen, für ein Aryl mit 6 bis 12 Kohlenstoffatomen, ein Aralkyl mit 7 bis 12 Kohlenstoffatomen stehen, oder $A^1$ $A^2$, $A^3A^4$, $A^5A^6$, $A^7A^8$ unabhängig voneinander, gleich oder verschieden sind und direkt oder über 0 oder N-$A^9$ miteinander zu einem Ring mit 3 bis 7 Ringgliedern verbunden sind, $A^9$ für ein Alkyl mit 1 bis 4 Kohlenstoffatomen steht und $B^-$ einen einwertigen Säurerest oder das Äquivalent eines mehrwertigen Säurerests bedeutet,

b) eine quartäre Ammoniumverbindung der Formel (II)

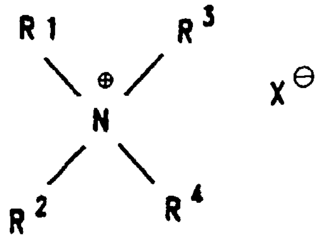

(II)

worin

$R^1$, $R^2$, $R^3$ und $R^4$ gleich oder verschieden sind und einen linearen oder verzweigten Alkoxypolyoxyalkyl-Rest der Formel $-(C_mH_{2m}O)_pR^5$ bedeuten, worin $R^5$ für einen linearen oder verzweigten Alkylrest mit 1 bis 16, vorzugsweise 1 bis 8, Kohlenstoffatomen oder für $C_1$-$C_4$-Alkyl-aryl, insbesondere Benzyl, steht, m eine ganze Zahl von 1 bis 10, vorzugsweise 1 bis 5, und p eine Zahl von 1 bis 15, vorzugsweise 2 bis 10, bedeuten; oder
einen linearen oder verzweigten Alkylrest mit 1 bis 30, vorzugsweise 1 bis 18, Kohlenstoffatomen;
oder einen unsubstituierten Phenyl- oder Naphthylrest; oder einen substituierten Phenyl- oder Naphthylrest, wobei die Substituenten die Bedeutung Halogen, $C_1$-$C_4$-Alkyl, $C_1$-$C_4$-Alkoxy, Nitro, $CF_3$ oder Cyano haben; und

$X^{\ominus}$ ein Anion, vorzugsweise F-, $HF_2$-, $Cl^-$, $I^-$, $Br^-$, $BF_4^-$, ½$SO_4^{2-}$, $C_6H_5$-$SO_3^-$, p-$CH_3$-$C_6H_4SO_3^-$, $HSO_4^-$, $PF_6^-$ oder $CF_3SO_3^-$, ist;

und

c) eine quartäre Phosphoniumverbindung der Formel (III)

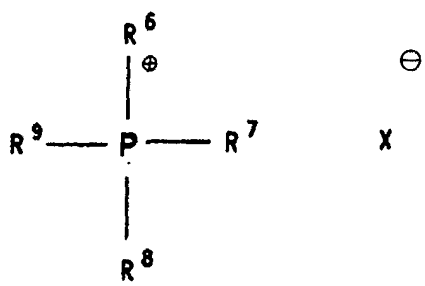

(III) ,

worin

$R^6$, $R^7$, $R^8$ und $R^9$ gleich oder verschieden sind und
einen linearen oder verzweigten Alkylrest mit 1 bis 22, vorzugsweise 1 bis 16, Kohlenstoffatomen; oder einen unsubstituierten oder substituierten Arylrest oder einen $C_1$-$C_4$-Alkyl-arylrest, wobei Aryl die Bedeutung Phenyl oder Naphthyl hat und die besagten Substituenten Halogen, $C_1$-$C_4$-Alkyl, $C_1$-$C_4$-Alkoxy, Nitro oder Cyano bedeuten;

$X^{\ominus}$ wie vorstehend definiert ist; und

d) ein Kronenether oder ein Polyether der Formel (IV)

$$R^{10}\text{-}(O\text{-}C_xH_{2x})_r\text{-}OR^{11} \quad \text{(IV)},$$

worin

| | |
|---|---|
| $R^{10}$ und $R^{11}$ | gleich oder verschieden sind und einen linearen oder verzweigten Alkylrest mit 1 bis 16, vorzugsweise 1 bis 8, Kohlenstoffatomen bedeuten; |
| x | eine ganze Zahl von 2 bis 6, vorzugsweise 2 bis 3, und |
| r | eine Zahl von 0 bis 20, vorzugsweise 1 bis 18, insbesondere 4 bis 14, ist. |

Der erfindungsgemäße Katalysator umfaßt alle denkbaren Kombinationen einer Verbindung gemäß a) mit einer Verbindung gemäß b) oder mit einer Verbindung gemäß c) oder mit einer Verbindung gemäß d) oder mit einem Gemisch aus b) und c), oder b) und d), oder c) und d), oder b), c) und d), wobei die genannten Verbindungen gemäß a) bis d) selbst jeweils ebenfalls eine Mischung von entsprechenden Verbindungen sein können.

Besonders bevorzugt ist ein Katalysator, bestehend aus Komponente a) und b), wobei mindestens einer der Reste $R^1$, $R^2$, $R^3$ und $R^4$ einen linearen oder verzweigten Alkoxypolyoxyalkyl-Rest der Formel $-(C_mH_{2m}O)_pR^5-$ bedeutet, oder bestehend aus Komponente a) und d).

Die Mischungsverhältnisse der Komponente a) mit den Komponenten b), c) und/oder d) können in weiten Grenzen schwanken, mit der Maßgabe, daß die Komponente a) 5 bis 95 Gew.-%, vorzugsweise 10 bis 80 Gew.-%, des gesamten Katalysators ausmacht.

Besonders bevorzugt ist ein Katalysator, bestehend aus den Komponenten a) und d), insbesondere im Verhältnis a): d) zwischen 2:1 bis 1:20, bevorzugt 1:1 bis 1:15, besonders bevorzugt 1:2 bis 1:10.

Komponente a):

Man kann eine Verbindung der Formel (I), worin $A^1$, $A^2$, $A^3$, $A^4$, $A^5$, $A^6$, $A^7$, $A^8$ unabhängig voneinander, gleich oder verschieden sind und für ein geradkettiges oder verzweigtes Alkyl oder Alkenyl, insbesondere Alkyl, mit 1 bis 12, insbesondere 1 bis 8, bevorzugt 1 bis 4 Kohlenstoffatomen, oder Cycloalkyl mit 4 bis 8, insbesondere 5 bis 6 Kohlenstoffatomen, stehen, einsetzen. Diese Verbindungen sind von besonderem Interesse, da sie sich auf vergleichsweise einfache Art ausgehend von den entsprechenden Dialkylaminen, Dialkenylaminen, Dicycloalkylaminen, sekundären Aminen, die einen Alkyl- und Alkenylrest, einen Alkyl- und Cycloalkylrest oder einen Alkenyl- und Cycloalkylrest enthalten, herstellen lassen.

Man kann eine Verbindung der Formel (I), worin $A^1A^2 = A^3A^4$ oder $A^1A^2 = A^3A^4 = A^5A^6$ oder $A^1A^2 = A^3A^4 = A^5A^6 = A^7A^8$ ist, einsetzen. Diese Verbindungen, in denen zwei oder mehrere der Gruppen $A^1A^2$, $A^3A^4$, $A^5A^6$ und $A^7A^8$ einander gleich sind, sind relativ gut zugänglich.

Als Beispiele für Alkyl sind Methyl, Ethyl, n-Propyl, i-Propyl, n-Butyl, i-Butyl, n-Pentyl, 3-Methylbutyl, n-Hexyl, 2-Ethylhexyl, insbesondere Methyl, Ethyl, n-Propyl, n-Butyl und als Beispiele für Alkenyl sind Allyl, Prop-(2)-enyl, n-But-(2)-enyl, und als Beispiele für Cycloalkyl sind Cyclopentyl, Cyclohexyl, 4-Methylcyclohexyl, 4-tert.-Butylcyclohexyl, zu nennen.

Man kann auch eine Verbindung der Formel (I), worin $A^1=A^2$, $A^3=A^4$, $A^5=A^6$ und/oder $A^7=A^8$ ist, einsetzen. Diese Verbindungen sind vergleichsweise leicht zugänglich und deshalb von Interesse.

Man kann auch eine Verbindung der Formel (I), worin $A^1 = A^2 = A^3 = A^4$ oder $A^1 = A^2 = A^3 = A^4 = A^5 = A^6$ oder $A^1 = A^2 = A^3 = A^4 = A^5 = A^6 = A^7 = A^8$ ist, einsetzen. Diese vorstehend genannten Verbindungen, in denen vier, sechs oder acht der Reste $A^1$ bis $A^8$ gleich sind, sind aufgrund ihrer leichten Zugänglichkeit ebenfalls von Interesse.

Es ist auch möglich, eine Verbindung der Formel (I), worin $A^1A^2$ oder $A^1A^2$ und $A^3$ $A^4$ oder $A^1A^2$ und $A^3A^4$ und $A^5A^6$ oder $A^1A^2$ und $A^3A^4$ und $A^5A^6$ und $A^7A^8$ direkt oder über O oder N-$A^9$ miteinander zu einem gesättigten oder ungesättigten Ring mit 5 oder 6 Ringgliedern verbunden sind, einzusetzen. Dementsprechend enthalten diese Verbindungen einen, zwei, drei oder vier der vorstehend erwähnten Ringe.

Es ist ferner möglich, eine Verbindung der Formel (I), worin $A^1A^2$ oder $A^1A^2$ und $A^3A^4$ oder $A^1A^2$ und $A^3A^4$ und $A^5A^6$ oder $A^1A^2$ und $A^3A^4$ und $A^5A^6$ und $A^7A^8$ zu einem Ring, der das N-Atom, an dem die jeweiligen Reste $A^1$ bis $A^8$ sitzen, gegebenenfalls O oder N-$A^9$ und $CH_2$-Gruppen als Ringglieder umfaßt, verbunden sind, einzusetzen. In dieser Stoffgruppe bilden das N-Atom mit den an ihnen jeweils befindlichen Resten $A^1$ bis $A^8$ beispielsweise einen Hexahydropyridinring, Tetrahydropyrrolring, einen Hexahydropyrazinring oder Morpholinring. Dementsprechend enthalten diese Verbindungen einen, zwei, drei oder vier der vorstehend erwähnten Ringe.

In der Verbindung der Formel (I) steht $B^-$, wie bereits eingangs erwähnt, für einen einwertigen Säurerest oder das Äquivalent eines mehrwertigen Säurerestes, insbesondere den Rest einer anorganischen Mineralsäure, einer organischen Carbonsäure, einer aliphatischen oder aromatischen Sulfonsäure.

Üblicherweise setzt man eine Verbindung der Formel (I), worin $B^-$ für $F^-$, $Cl^-$, $Br^-$, $J^-$, $HF_2^-$, $BF_4^-$, $C_6H_5SO_3^-$, p-$CH_3$-$C_6H_5SO_3^-$, $HSO_4^-$, $PF_6^-$, $CF_3SO_3^-$, insbesondere für $F^-$, $Cl^-$, $Br^-$, $J^-$, $HF_2^-$, $BF_4^-$, steht, ein.

Ohne Anspruch auf Vollständigkeit zu erheben, seien als Beispiele für Verbindungen der Formel (I) genannt:

Tetrakis(dimethylamino)phosphoniumchlorid
Tetrakis(diethylamino)phosphoniumchlorid

Tetrakis(dimethylamino)phosphoniumbromid
Tetrakis(diethylamino)phosphoniumbromid
Tetrakis(dipropylamino)phosphoniumchlorid oder -bromid
Tris(diethylamino)(dimethylamino)phosphoniumchlorid oder -bromid
Tetrakis(dibutylamino)phosphoniumchlorid oder -bromid
Tris(dimethylamino)(diethylamino)phosphoniumchlorid oder -bromid
Tris(dimethylamino)(cyclopentylamino)phosphoniumchlorid oder -bromid
Tris(dimethylamino)(dipropylamino)phosphoniumchlorid oder -bromid
Tris(dimethylamino)(dibutylamino)phosphoniumchlorid oder -bromid
Tris(dimethylamino)(cyclohexylamino)phosphoniumchlorid oder -bromid
Tris(dimethylamino)(diallylamino)phosphoniumchlorid oder -bromid
Tris(dimethylamino)(dihexylamino)phosphoniumchlorid oder -bromid
Tris(diethylamino)(dihexylamino)phosphoniumchlorid oder -bromid
Tris(dimethylamino)(diheptylamino)phosphoniumchlorid oder -bromid
Tris(diethylamino)(diheptylamino)phosphonium chlorid oder -bromid
Tetrakis(pyrrolidino)phosphoniumchlorid oder -bromid
Tetrakis(piperidino)phosphoniumchlorid oder -bromid
Tetrakis(morpholino)phosphoniumchlorid oder -bromid
Tris(piperidino)(diallylamino)phosphoniumchlorid oder -bromid
Tris(pyrrolidino)(ethylmethylamino)phosphoniumchlorid oder -bromid
Tris(pyrrolidino)(diethylamino)phosphoniumchlorid oder -bromid.

Man kann auch ein Gemisch zweier oder mehrerer Verbindungen der Formel (I) verwenden. Besonders einfach gestaltet sich dies, wenn man Gemische von Verbindungen der Formel (I) verwendet.

Die Verbindungen der Formel (I) lassen sich beispielsweise durch Umsetzung von Phosphorpentachlorid mit Dialkylaminen herstellen. Aus der nachfolgenden Gleichung ist die Umsetzung unter Verwendung von Dimethylamin zu entnehmen:

$$PCl_5 + HN(CH_3)_2 \rightarrow P[N(CH_3)_2]_4\ Cl$$

Man kann aber auch Phosphorpentachlorid stufenweise mit unterschiedlichen sekundären Aminen, beispielsweise Dialkylaminen, zur Reaktion bringen, um unsymmetrisch substituierte Verbindungen der Formel (I) zu erhalten. Weitere Möglichkeiten, Verbindungen der Formel (I) zu synthetisieren, sind von R. Schwesinger et al., Angew. Chem. 103 (1991) 1376 und R. Schwesinger et al., Chem. Ber. 127 (1994) 2435 bis 2454 beschrieben.

Komponente b):

In dem in der Verbindung der Formel (II) enthaltenen linearen oder verzweigten Alkoxypolyoxyalkyl-Rest der Formel $-(C_mH_{2m}O)_pR^5$ können gleiche oder unterschiedliche Alkoxy-Einheiten miteinander verknüpft sein.
Die Anzahl der in der Verbindung der Formel (II) enthaltenen linearen oder verzweigten Alkoxypolyoxyalkyl-Reste beträgt vorzugsweise 1 oder 2.
Besonders bevorzugte Verbindungen der Formel (II) im Sinne der vorliegenden Erfindung sind Dimethyl-di(ethoxypolyoxypropyl)-ammoniumchlorid, Dimethyldi(ethoxypolyoxypropylmethylether)-ammoniumchlorid, Dimethyl(ethoxypolyoxypropyl)-(ethoxypolyoxypropylmethylether)-ammoniumchlorid, Dimethyl-di(ethoxypolyoxyethyl)-ammoniumchlorid, Dimethyldi(ethoxypolyoxyethylmethylether)-ammoniumchlorid, Dimethyl(ethoxypolyoxyethyl)-(ethoxypolyoxyethylmethylether)-ammoniumchlorid, jeweils mit einer mittleren Kettenlänge p von 3, weiterhin Trimethyl-(ethoxypolyoxypropyl)-ammoniumchlorid und Trimethyl-(ethoxypolyoxypropylmethylether)-ammoniumchlorid, jeweils mit einer mittleren Kettenlänge p von 8, oder ein Gemisch der genannten Verbindungen.

Die beschriebenen Verbindungen der Formel (II) lassen sich auf bekannte Weise (US-PS 3 123 641; US-PS 3 141 905) aus den entsprechenden Ethanolaminen herstellen, die nach Umsetzung mit Alkylenoxiden und anschließender Quaternisierung mit oder ohne gleichzeitiger Veretherung in guten Ausbeuten die gewünschten Verbindungen liefern.

Bevorzugte Verbindungen der Formel (II) sind Octadecyltrimethylammoniumchlorid, Distearyldimethylammoniumchlorid, Tetramethylammoniumchlorid, Tetramethylammoniumbromid, Hexadecyltrimethylammoniumchlorid und Benzyltrimethylammoniumchlorid.

Komponente c):

Bevorzugte Verbindungen der Formel (III) im Sinne der vorliegenden Erfindung sind Hexadecyltributylphosphoniumbromid, Stearyltributylphosphoniumbromid, Tetrabutylphosphoniumchlorid, Tetrabutylphosphoniumbromid, Tetraoctylphosphoniumbromid, Tetraphenylphosphoniumbromid und -chlorid.

Komponente d):

Bevorzugte Polyether der Formel (IV) im Sinne der vorliegenden Erfindung besitzen eine mittlere Molmasse zwischen 300 und 800 g/mol. Besonders bevorzugt ist ein Gemisch von Polyethylenglykoldimethylethern der Kettenlängen r von 6 bis 17 und einer mittleren Molmasse von 500 g/mol. Anstelle von oder in Kombination mit Polyethern der Formel (IV) können auch Kronenether, beispielsweise 18-Krone-6, Dibenzo-18-krone-6, Benzo-18-krone-6, 15-Krone-5, Benzo-15-krone-5, Decyl-18-krone-6 und Dicyclohexyl-18-krone-6, eingesetzt werden.

Es ist als überraschend anzusehen, daß der erfindungsgemäße Katalysator zu einer starken Beschleunigung der Reaktion führt, wodurch die Halogen-Fluor-Austauschreaktion (Halex-Reaktion) unter erheblich schonenderen Bedingungen, insbesondere niedrigeren Temperaturen und/oder kürzeren Reaktionszeiten durchgeführt werden kann. Dadurch kann zugleich auch die Bildung unerwünschter Nebenprodukte zurückgedrängt oder weitgehend vermieden werden.

Gegenstand der vorliegenden Erfindung ist daher auch die Verwendung des beschriebenen Katalysators für Halex-Reaktionen, dadurch gekennzeichnet, daß man eine Verbindung, die gegen Fluor austauschbares Halogen enthält, mit einem Fluorid oder einem Gemisch von Fluoriden der allgemeinen Formel (V)

$$MeF \qquad (V),$$

worin Me für ein stöchiometrisches Equivalent eines Erdalkalimetallions, ein Alkalimetallion oder ein Tetraalkylammonium-Ion, wobei Alkyl bevorzugt 1 bis 4 Kohlenstoffatome hat, steht, in Gegenwart des besagten Katalysators, in Gegenwart oder Abwesenheit eines Lösungsmittels bei einer Temperatur von 40 bis 260°C umsetzt.

Unter dem Begriff gegen Fluor austauschbares Halogen wird Chlor, Brom oder Jod, insbesondere Chlor oder Brom, bevorzugt Chlor, das durch Fluorid im Sinne einer nucleophilen Substitution ausgetauscht werden kann, verstanden.

Man setzt den erfindungsgemäßen Katalysator in einer Menge von 0,5 bis 35 Gew.-%, insbesondere 1 bis 30 Gew.-%, bevorzugt 3 bis 25 Gew.-%, bezogen auf die Verbindung, die gegen Fluor austauschbares Halogen enthält, ein.

Ein weiterer Vorteil des erfindungsgemäßen Katalysators besteht darin, daß man eine Vielzahl von Verbindungen als Ausgangsmaterial für die Halex-Reaktion einsetzen kann.

So ist es möglich, als Verbindung, die gegen Fluor austauschbares Halogen enthält, eine am Kern einen gegen Fluor austauschbaren Chlor- oder Bromsubstituenten, insbesondere Chlorsubstituenten, besitzende aromatische Verbindung mit 0 bis 3 Stickstoffatomen im Kern, die gegebenenfalls wenigstens einen weiteren, eine nucleophile Substitution der aromatischen Verbindung begünstigenden Substituenten aufweist, einzusetzen.

Ohne Anspruch auf Vollständigkeit zu erheben, kommen als Ausgangsverbindungen für das erfindungsgemäße Verfahren aromatische Verbindungen vom Benzol-, Naphthalin-, Pyridin-, Anthracen-, Phenanthren-, Pyrimidin- und Pyrazin-Typ sowie vom Typ benzoannellierter Ringsysteme des Pyridins (Chinolin-, Isochinolin-, Acridin-, Acridon-Typ), des Pyrimidins, Pyrazins und Piperazins (Benzodiazine des Cinnolin-, Phthalazin-, Chinazolin-, Chinoxalin-, Phenazin-, Phenoxazin-Typs) und deren Derivate in Betracht, die gegebenfalls wenigstens einen weiteren, die nucleophile Substitution der aromatischen Verbindung begünstigenden Substituenten aufweisen. Dieser weitere, die nucleophile Substitution der aromatischen Verbindung begünstigende Substituent führt üblicherweise zu einer Aktivierung der aromatischen Verbindung, die dadurch einer Halogen-Fluor-Austauschreaktion leichter zugänglich wird.

Bei dem weiteren, die nucleophile Substitution an der aromatischen Verbindung begünstigenden Substituenten handelt es sich um -J- und -M-Substituenten, die die Elektronendichte des Aromaten herabsetzen und dadurch eine elektrophile Substitution erschweren. Der Aromat wird dadurch jedoch gegenüber einer nucleophilen Substitution aktiviert. Die aktivierende Wirkung dieser Substituenten ist besonders groß, wenn sie in ortho- oder para-Stellung zu dem gegen Fluor auszutauschenden Halogen, insbesondere Chlor oder Brom, bevorzugt Chlor, stehen.

Ohne Anspruch auf Vollständigkeit zu erheben, seien als weitere, die nucleophile Substitution und damit die Halogen-Fluor-Austauschreaktion, insbesondere die Chlor-Fluor-Austauschreaktion begünstigende Substituenten F, Cl, Br, J, $NO_2$, NO, $CF_3$, CN, CHO, COF, COCl, $SO_2F$, $SO_2Cl$, $OCF_3$, $SCF_3$, $SOCF_3$, $SO_2CF_3$, COOR, CONRR', $SO_2R$, COR, OR oder ein Rest -CO-O-CO-, -CO-NR-CO-, der zwei ortho-Stellungen verknüpft, insbesondere F, Cl, $NO_2$, $CF_3$, CN, CHO, COCl, $SO_2Cl$, COOR, $SO_2CF_3$, CONRR', $SO_2R$, COR, bevorzugt F, Cl, $NO_2$, $CF_3$, CN, CHO, COCl, wobei

R und R' jeweils unabhängig voneinander, gleich oder verschieden sind, und für H, ein geradkettiges oder verzweigtes Alkyl mit 1 bis 6, insbesondere 1 bis 4 Kohlenstoffatomen, ein Aryl mit 6 bis 12 Kohlenstoffatomen oder Aralkyl mit 7 bis 12 Kohlenstoffatomen und die Alkyle und Aralkyle, gegebenenfalls einfach bis dreifach halogensubstituiert, insbesondere fluoriert oder chloriert, sind, genannt.

Man kann eine am Kern einen gegen Fluor austauschbaren Chlor- oder Bromsubstituenten, insbesondere Chlorsubstituenten, besitzende aromatische Verbindung einsetzen, die wenigstens einen weiteren Substituenten aus der Reihe F, Cl, Br, J, $NO_2$, $CF_3$, CN, CHO, COF, COCl, $SO_2F$, $SO_2Cl$, $OCF_3$, $SCF_3$, $SOCF_3$, $SO_2CF_3$, COOR, CONRR', $SO_2R$, COR oder OR oder einen Rest -CO-O-CO-, -CO-NR-CO-, der zwei ortho-Stellungen verknüpft, aufweist, wobei R und R' jeweils unabhängig voneinander, gleich oder verschieden sind, und für H, ein geradkettiges oder verzweigtes Alkyl mit 1 bis 6 Kohlenstoffatomen, ein Aryl mit 6 bis 12 Kohlenstoffatomen oder Aralkyl mit 7 bis 12 Kohlenstoffatomen stehen und die Alkyl und Aralkyle gegebenenfalls einfach bis dreifach halogensubstituiert sind.

Die vorstehend erwähnten aromatischen Verbindungen können auch zusätzliche Substituenten enthalten, beispielsweise Alkylreste, Aminogruppen, Alkylaminogruppen, Hydroxygruppen oder Alkoxygruppen.

Man kann als Ausgangsmaterial eine am Kern einen gegen Fluor austauschbaren Chlor- oder Bromsubstituenten, insbesondere Chlorsubstituenten, besitzende aromatische Verbindung, die wenigstens ein gegen Fluor austauschbares Chlor oder Brom, insbesondere Chlor, als weiteren Substituenten und gegebenenfalls wenigstens einen weiteren Substituenten aus der Reihe F, $NO_2$, $CF_3$, CN, CHO, COF, COCl, $SO_2F$, $SO_2Cl$, $OCF_3$, $SCF_3$, $SO_2CF_3$, COOR, CONRR', $SO_2R$, COR, OR, -CO-O-CO- oder -CO-NR-CO- besitzt, einsetzen. Diese Ausgangsverbindungen besitzen demzufolge wenigstens zwei gegen Fluor austauschbare Halogensubstituenten, die unabhängig voneinander für Chlor oder Brom, insbesondere Chlor, stehen können. Diese Verbindungen sind üblicherweise einem einfachen oder zweifachen Halogen-Fluor-Austausch zugänglich, ohne daß sie einen weiteren Substituenten aus der vorstehend genannten Reihe besitzen müssen. Sie können aber auch einen weiteren, die nucleophile Substitution der aromatischen Verbindung begünstigenden Substituenten aus der Reihe der zuvor genannten Reste besitzen. Die Anwesenheit der Substituenten erhöht die Reaktivität der aromatischen Verbindung hinsichtlich der Halogen-Fluor-Austauschreaktion.

Mit gutem Erfolg kann man in das erfindungsgemäße Verfahren eine Verbindung der allgemeinen Formel (VI)

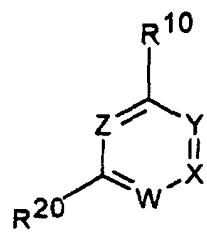

(VI)

worin W für N oder C-$R^{30}$, X für N oder C-$R^{40}$, Y für N oder C-$R^{50}$, Z für N oder C-$R^{60}$ steht, W, X und Y nicht zugleich N sind, $R^{10}$, $R^{20}$, $R^{30}$, $R^{40}$, $R^{50}$, $R^{60}$ gleich oder verschieden sind und H, F, Cl, Br, J, $NO_2$, NO, $CF_3$, CN, CHO, COF, COCl, $SO_2F$, $SO_2Cl$, $OCF_3$, $SCF_3$, $SO_2CF_3$, COOR, CONRR', $SO_2R$, COR, OR, einen Rest -CO-O-CO, -CO-NR-CO- oder -CR"=CR"-CR"=CR"-, der zwei ortho-Stellungen verknüpft, bedeuten, R und R' die vorstehende Bedeutung besitzen und R" unabhängig voneinander, gleich oder verschieden sind und dieselbe Bedeutung wie $R^{10}$ bis $R^{60}$ haben, und wenigstens einer der Reste $R^{10}$ bis $R^{60}$ Chlor oder Brom, insbesondere Chlor ist, einsetzen.

Man kann eine Verbindung der Formel (VI), worin $R^{10}$, $R^{20}$, $R^{30}$, $R^{40}$, $R^{50}$, $R^{60}$ gleich oder verschieden sind und insbesondere H, F, Cl, Br, $NO_2$, $CF_3$, CN, CHO, COCl, bevorzugt H, F, Cl, $NO_2$, CN, CHO bedeuten, einsetzen.

Man kann auch eine Verbindung der Formel (VI), worin nur einer der Reste $R^{10}$ bis $R^{60}$ Chlor oder Brom, insbesondere Chlor, ist, keiner der Reste W, X, Y, Z für ein Stickstoffatom steht und wenigstens einer der verbleibenden Reste aus der Gruppe $R^{10}$ bis $R^{60}$ $NO_2$, $CF_3$, CN, CHO, COF, COCl, $SO_2F$, $SO_2Cl$, $OCF_3$, $SCF_3$, $SO_2CF_3$, COOR, CONRR', $SO_2R$, COR, OR, -CO-O-CO-, -CO-NR-CO- oder -CR"=CR"-CR"=CR"- ist, einsetzen.

Man kann in die Halex-Reaktion eine Verbindung der Formel (VI), worin 2 oder mehrere der Reste $R^{10}$ bis $R^{60}$ Chlor oder Brom, insbesondere Chlor, sind, die Reste W, X, Y, Z für 0 bis 3 Stickstoffatome stehen und die verbleibenden Reste aus der Gruppe $R^{10}$ bis $R^{60}$ alle Wasserstoff sein können, einsetzen.

Man kann in das Verfahren auch eine Verbindung der Formel (VI), worin nur einer der Reste $R^{10}$ bis $R^{60}$ Chlor oder Brom, insbesondere Chlor, ist, wenigstens einer der Reste W, X, Y, Z für ein Stickstoffatom steht und die verbleibenden Reste aus der Gruppe $R^{10}$ bis $R^{60}$ alle Wasserstoff sein können, einsetzen.

Der Einbau wenigstens eines Stickstoffatomes in den aromatischen Ring erhöht die Reaktivität der aromatischen Verbindung derart, daß gegebenenfalls auch ohne Anwesenheit eines weiteren, die nucleophile Substitution der aromatischen Verbindung begünstigenden Substituenten ein Halogen-Fluor-Austausch stattfinden kann.

Mit gutem Erfolg kann man eine Verbindung der allgemeinen Formel (VII)

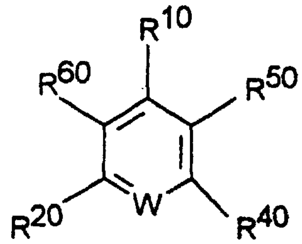

( VII )

worin W für N oder C-$R^{30}$ steht, einer der Reste $R^{10}$, $R^{20}$, $R^{40}$, $R^{50}$, $R^{60}$ und gegebenenfalls $R^{30}$, Cl, F, $NO_2$, $CF_3$, CN, CHO, COF, COCl, $SO_2F$, $SO_2Cl$, $OCF_3$, $SCF_3$, $SO_2CF_3$, COOR, CONRR', $SO_2R$, COR oder OR ist oder zwei der Reste, die in ortho-Stellung zueinander stehen, -CO-O-CO- oder -CO-NR-CO- bedeuten, wobei R und R' jeweils unabhängig voneinander, gleich oder verschieden sind und für H, ein geradkettiges oder verzweigtes Alkyl mit 1 bis 6 Kohlenstoffatomen, ein Aryl mit 6 bis 12 Kohlenstoffatomen oder Aralkyl mit 7 bis 12 Kohlenstoffatomen stehen, ein weiterer der Reste $R^{10}$, $R^{20}$, $R^{40}$, $R^{50}$, $R^{60}$ und gegebenenfalls $R^{30}$ Cl ist und die übrigen Reste für H, F oder Cl stehen, einsetzen.

Mit guter Aussicht auf Erfolg kann man auch eine Verbindung der allgemeinen Formel (VII), worin W für N oder C-$R^{30}$ steht, einer der Reste $R^{10}$, $R^{20}$, $R^{40}$, $R^{50}$, $R^{60}$ oder der Rest $R^{30}$, Cl, F, $NO_2$, $CF_3$, CN, CHO, COF, COCl, $SO_2F$, $SO_2Cl$, $OCF_3$, $SCF_3$, $SO_2CF_3$, COOR, CONRR', $SO_2R$, COR oder OR ist oder zwei der Reste, die in ortho-Stellung zueinander stehen, -CO-O-CO- oder -CO-NR-CObedeuten, wobei R und R' jeweils unabhängig voneinander, gleich oder verschieden sind und für H, ein geradkettiges oder verzweigtes Alkyl mit 1 bis 6 Kohlenstoffatomen, ein Aryl mit 6 bis 12 Kohlenstoffatomen oder Aralkyl mit 7 bis 12 Kohlenstoffatomen stehen, ein weiterer der Reste $R^{10}$, $R^{20}$, $R^{40}$, $R^{50}$, $R^{60}$ Cl ist und die übrigen Reste für H, F oder Cl stehen, einsetzen.

Die Reste -CO-O-CO- und -CO-NR-CO- betreffen allgemein zwei der Reste $R^{10}$ bis $R^{60}$, die in ortho-Stellung zueinander stehen, insbesondere zwei in ortho-Stellung zueinander stehende Reste aus der Gruppe $R^{10}$, $R^{20}$, $R^{40}$, $R^{50}$ und $R^{60}$, falls W für N steht, oder zwei in ortho-Stellung zueinander stehende Reste aus der Gruppe $R^{20}$, $R^{30}$ und $R^{40}$, falls W für C-$R^{30}$ steht.

In der Verbindung der Formel (VII) steht einer der Reste $R^{10}$, $R^{20}$, $R^{40}$, $R^{50}$, $R^{60}$ und gegebenenfalls $R^{30}$ oder der Rest $R^{30}$ insbesondere für Cl, F, $NO_2$, $CF_3$, CN, CHO, COF, COCl, $OCF_3$, COOR, COONRR', COR, OR, -CO-O-CO- oder -CO-NR-CO-, bevorzugt für Cl, F, $NO_2$, $CF_3$, CN, CHO, COOR oder COCl, R und R' bedeuten insbesondere H, ein geradkettiges oder verzweigtes Alkyl mit 1 bis 4 Kohlenstoffatomen oder Aryl mit 6 bis 12 Kohlenstoffatomen, bevorzugt H oder ein geradkettiges oder verzweigtes Alkyl mit 1 bis 3 Kohlenstoffatomen, besonders bevorzugt Methyl oder Ethyl, einer oder zwei der Reste $R^{10}$, $R^{20}$, $R^{40}$, $R^{50}$, $R^{60}$ und gegebenenfalls $R^{30}$ stehen für Cl und die übrigen Reste sind gleich oder verschieden und stehen für H oder Cl.

Die vorstehend aufgeführte Formel (VII) umfaßt nichtaktivierte Verbindungen, in denen einer der Reste $R^{10}$, $R^{20}$, $R^{40}$, $R^{50}$, $R^{60}$ und gegebenenfalls $R^{30}$ für Cl oder F steht und zusätzlich einer, zwei oder mehrere der Reste $R^{10}$, $R^{20}$, $R^{40}$, $R^{50}$, $R^{60}$ und gegebenenfalls $R^{30}$ für Cl stehen und die daraus resultierenden Verbindungen ein, zwei oder mehr Cl enthalten, falls der eine der vorstehend genannten Reste F ist, oder zwei, drei oder mehr Cl enthalten, falls der eine der vorstehend genannten Reste nicht F sondern Cl ist.

Beispiele für derartige nichtaktivierte Derivate des Pyridins, wobei in Formel (VII) W für N steht, sind 2,3-Dichlorpyridin, 2,4-Dichlorpyridin, 2,5-Dichlorpyridin, 2,6-Dichlorpyridin, 3,4-Dichlorpyridin, 3,5-Dichlorpyridin, 2,3,4-Trichlorpyridin, 2,3,5-Trichlorpyridin, 2,3,6-Trichlorpyridin, 2,4,6-Trichlorpyridin, Tetrachlorpyridin und Pentachlorpyridin sowie fluorierte Chlorpyridine, die sich infolge Teilfluorierung aus den vorstehend genannten Chlorpyridinen bilden.

Beispiele für derartige nichtaktivierte Derivate des Benzols, wobei in Formel (VII) W für C-$R^{30}$ steht, sind 1,2-Dichlorbenzol, 1,3-Dichlorbenzol, 1,4-Dichlorbenzol, 1,2,3-Trichlorbenzol, 1,2,4-Trichlorbenzol, 1,3,5-Trichlorbenzol, 1,2,3,4-Tetrachlorbenzol, 1,2,3,5-Tetrachlorbenzol, 1,2,4,5-Tetrachlorbenzol, aber auch fluorierte Chlorbenzole, die sich infolge Teilfluorierung aus den vorstehend genannten Chlorbenzolen bilden.

Die vorstehend aufgeführte Formel (VII) umfaßt auch Verbindungen, die einen aktivierenden Rest enthalten. Als aktivierender Rest kommen $NO_2$, $CF_3$, CN, CHO, COF, COCl, $SO_2F$, $SO_2Cl$, $OCF_3$, $SCF_3$, $SO_2CF_3$, COOR, COONRR', $SO_2R$, COR, OR, -CO-O-CO- oder -CO-NR-CO-, insbesondere $NO_2$, $CF_3$, CN, CHO, COF, COCl, $OCF_3$, COOR, CONRR', COR, OR, -CO-O-CO- oder -CO-NR-CO-, bevorzugt $NO_2$, $CF_3$, CN, CHO, COCl, COOR, COR in Betracht.

Bei den Verbindungen, die einen aktivierenden Rest enthalten, ist einer der Reste $R^{10}$ bis $R^{60}$ in Formel (VII), insbesondere einer der Reste aus der Gruppe $R^{10}$, $R^{20}$, $R^{40}$, $R^{50}$, $R^{60}$, falls W für N steht, oder insbesondere der Rest $R^{30}$, falls W für C-$R^{30}$ steht, der aktivierende Rest. Der aktivierende Rest entfaltet eine besonders große Wirkung, wenn das gegen F auszutauschende Cl in ortho- oder para-Stellung zu dem aktivierenden Rest steht. In diesem Zusammenhang sei nochmals erwähnt, daß das N-Atom im Pyridinring ebenfalls im Sinne eines Chlor-Fluor-Austausches aktivierend wirkt.

Das erfindungsgemäße Verfahren betrifft nicht nur den Austausch von Cl in ortho-Stellung und/oder para-Stellung zu einem aktivierenden Rest, sondern auch den Austausch von Cl in den weniger begünstigten meta-Stellungen. So kann man auch Verbindungen der allgemeinen Formel (VIII),

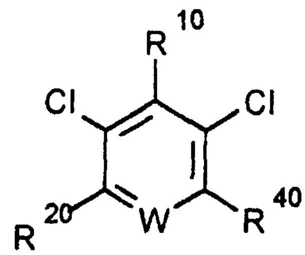

(VIII)

worin W für N oder C-$R^{30}$ steht, wobei $R^{30}$ $NO_2$, $CF_3$, CN, CHO, COF, COCl, $SO_2F$, $SO_2Cl$, $OCF_3$, $SCF_3$, $SO_2CF_3$, COOR, COONRR', $SO_2R$, COR, OR ist oder zwei in ortho-Stellung stehende Reste aus der Gruppe $R^{20}$, $R^{30}$, $R^{40}$ -CO-O-CO- oder -CO-NR-CO- sind, insbesondere $NO_2$, $CF_3$, CN, CHO, COF, COCl, $OCF_3$, COOR, CONRR', COR, OR ist oder zwei in ortho-Stellung stehende Reste aus der Gruppe $R^{20}$, $R^{30}$, $R^{40}$ -CO-O-CO- oder -CO-NR-CO- sind, bevorzugt $NO_2$, $CF_3$, CN, CHO, COCl und $R^{10}$, $R^{20}$, $R^{40}$ für H, F oder Cl stehen, einsetzen.

Ohne Anspruch auf Vollständigkeit zu erheben, seien folgende Stoffe, die gegen Fluor austauschbares Halogen enthalten, als kleine Auswahl genannt: 4-Nitrochlorbenzol, 2-Chlornitrobenzol, 2,4-Dichlornitrobenzol, 2-Chlorbenzaldehyd, 4-Chlorbenzaldehyd, 2-Chlorbenzonitril, 4-Chlorbenzonitril, 2-Chlorbenzoylchlorid, 4-Chlorbenzoylchlorid, 2,4-Dichlorbenzaldehyd, 2,6-Dichlorbenzaldehyd, 2,4-Dichlorbenzonitril, 2,6-Dichlorbenzonitril, 2,4-Dichlorbenzoylchlorid, 2,6-Dichlorbenzoylchlorid und 1,3,5-Trichlorbenzol.

Man setzt als Fluorid der Formel (V) Calciumfluorid, Ammoniumfluorid, Lithiumfluorid, Natriumfluorid, Kaliumfluorid, Rubidiumfluorid, Cäsiumfluorid oder ein Gemisch derselben, insbesondere Lithiumfluorid, Natriumfluorid, Kaliumfluorid, Rubidiumfluorid, Cäsiumfluorid oder ein Gemisch derselben, bevorzugt Natriumfluorid, Kaliumfluorid, Cäsiumfluorid oder ein Gemisch derselben, besonders bevorzugt Kaliumfluorid, Cäsiumfluorid oder ein Gemisch derselben ein. Häufig genügt es, Kaliumfluorid allein einzusetzen.

Was das Mengenverhältnis Fluorid zu Ausgangsverbindungen anbelangt, so ist zu berücksichtigen, daß es Fälle geben kann, in denen ein Überschuß an Fluorid zu unerwünschten Nebenreaktionen führen kann. In diesen Fällen empfiehlt es sich, das Fluorid auch im Unterschuß einzusetzen. Man setzt üblicherweise das Fluorid der Formel (V):Äquivalent auszutauschendes Halogen im Verhältnis (0,5 bis 10):1, insbesondere (0,8 bis 5):1, bevorzugt (1 bis 2):1, besonders bevorzugt (1 bis 1,5):1 ein.

Die erfindungsgemäß durchgeführte Halex-Reaktion läßt sich in Anwesenheit oder Abwesenheit eines Lösungsmittels durchführen. Werden Lösungsmittel verwendet, so sind sowohl dipolar aprotische und aprotische als auch protische Lösungsmittel geeignet. Geeignete dipolar aprotische Lösungsmittel sind beispielsweise Dimethylsulfoxid (DMSO), Dimethylsulfon, Sulfolan, Dimethylformamid (DMFA), Dimethylacetamid, 1,3-Dimethylimidazolin-2-on, N-Methylpyrrolidon, Diglyme, Hexamethylphosphorsäuretriamid, Acetonitril und Benzonitril.
Diese Lösungsmittel können auch als Gemisch Anwendung finden.

Geeignete aprotische Lösungsmittel ohne ausgeprägten dipolaren Charakter sind aromatische Kohlenwasserstoffe oder chlorierte aromatische Kohlenwasserstoffe, beispielsweise Benzol, Toluol, ortho-, meta-, para-Xylol, technische Gemische isomerer Xylole, Ethylbenzol, Mesitylen, ortho-, meta-, para-Chlortoluol, Chlorbenzol und ortho-, meta-, para-Dichlorbenzol. Es können auch Gemische dieser Lösungsmittel verwendet werden.

Das aprotische oder dipolar aprotische Lösungsmittel kann in beliebigen Mengen, beispielsweise 5 bis 500 Gew.-%, verwendet werden, bevorzugt werden allerdings geringe Mengen im Bereich von 5 bis 30 Gew.-%, bezogen auf die Verbindung, die gegen Fluor austauschbares Halogen enthält. Bei der Verwendung von protischen Lösungsmitteln liegen die eingesetzten Mengen im Bereich von 0,1 bis 5 Gew.-%, bevorzugt 0,1 bis 2 Gew.-%, bezogen auf die Verbindung, die gegen Fluor austauschbares Halogen enthält.

Die Reaktionstemperatur hängt auch von der Art der Verbindung, die gegen Fluor austauschbares Halogen enthält, ab. So erfordern vergleichsweise reaktionsträge Verbindungen in der Regel höhere Reaktionstemperaturen, während vergleichsweise reaktive Ausgangsstoffe sich bereits bei relativ niedrigen Temperaturen mit Erfolg umsetzen lassen.

Gleiches trifft auch auf die Reaktionszeiten zu. Reaktionsträge Ausgangsstoffe erfordern in der Regel längere Reaktionszeiten als reaktivere Ausgangsstoffe.

An dieser Stelle sei darauf aufmerksam gemacht, daß ein Austausch lediglich eines Halogens gegen Fluor in der Regel einfacher durchzuführen ist, als ein Austausch von zwei oder mehreren Halogenen gegen Fluor. Der

zweifache oder mehrfache Halogen-Fluor-Austausch erfordert, sofern er überhaupt abläuft, üblicherweise erheblich schärfere Reaktionsbedingungen (höhere Reaktionstemperaturen und längere Reaktionszeiten) als ein einfacher Halogen-Fluor-Austausch.

In einer Vielzahl von Fällen genügt es, das erfindungsgemäße Verfahren bei einer Temperatur von 60 bis 250, insbesondere 90 bis 220, bevorzugt 120 bis 200°C, durchzuführen.

Die erfindungsgemäß durchgeführte Halex-Reaktion kann sowohl unter reduziertem Druck als auch unter Atmosphärendruck oder Überdruck ausgeübt werden. Diese Möglichkeit wird beispielsweise genutzt, indem geringe Mengen eines leicht siedenden aprotischen Lösungsmittels, das mit Wasser ein Azeotrop bildet, beispielsweise Benzol, Xylol, Mesitylen, Cyclohexan oder Toluol, vor Beginn der Reaktion in die Reaktionssuspension gegeben werden. Anschließend wird ein Teil des Lösungsmittels durch Anlegen eines reduzierten Druckes zusammen mit Wasser aus der Reaktionssuspension wieder entfernt. Durch diese Verfahrensweise läßt sich die Reaktionsgeschwindigkeit und die Ausbeute steigern und darüberhinaus die Bildung von Nebenprodukten minimieren.

Der erfindungsgemäße Katalysator kann in Abwesenheit oder Anwesenheit von Luftsauerstoff verwendet werden. Bevorzugt wird das Arbeiten unter Schutzgas, beispielsweise Argon oder Stickstoff.

Bei Durchführung des Verfahrens ist zu gewährleisten, daß während der gesamten Reaktion das Reaktionsgemisch gut durchmischt wird.

Das Verfahren läßt sich diskontinuierlich oder kontinuierlich durchführen.

Die nachfolgenden Beispiele belegen die Erfindung ohne sie zu beschränken

## Experimenteller Teil

### Herstellung von 4-Nitrofluorbenzol

### Beispiel 1

Herstellung von 4-Nitrofluorbenzol durch Umsetzung von 4-Nitrochlorbenzol mittels Tetrakis(diethylamino) phosphoniumbromid und Polyethylenglykoldimethylether (500 g/mol) als Katalysator.

Man legt in einem 1,5 l Vierhalskolben, der mit Thermometer, Ankerrührer und Rückflußkühler mit Blasenzähler bestückt ist, 157 g (1 mol) 4-Nitrochlorbenzol, 62,7 g (1,1 mol) Kaliumfluorid und 3,99 g (0,01 mol) Tetrakis (diethylamino)-phosphoniumbromid und 40 g (0,08 mol) Polyethylenglykoldimethylether (500) als Katalysator vor. Anschließend erhitzt man unter Rühren auf die vorgegebene Reaktionstemperatur und läßt der vorgegebenen Zeit entsprechend reagieren. Nach Beendigung der Umsetzung läßt man abkühlen, löst das Reaktionsgemisch in Chlorbenzol, filtriert unlösliche Bestandteile (Salze wie KCl, KF) ab und reinigt das Wertprodukt (4-Nitrofluorbenzol) durch fraktionierte Destillation unter reduziertem Druck.

### Vergleichsbeispiel 1

Herstellung von 4-Nitrofluorbenzol durch Umsetzung von 4-Nitrochlorbenzol mittels Tetrakis(diethylamino)phosphoniumbromid als Katalysator

Man setzt 157 g (1 mol) 4-Nitrochlorbenzol, 62,7 g (1,1 mol) Kaliumfluorid, jedoch 3,99 g (0,01 mol) Tetrakis (diethylamino)phosphoniumbromid ein und arbeitet wie in Beispiel 1 beschrieben.

### Vergleichsbeispiel 2

Herstellung von 4-Nitrofluorbenzol durch Umsetzung von 4-Nitrochlorbenzol mittels Polyethylenglykoldimethylether (500) als Katalysator.

Man setzt 157 g (1 mol) 4-Nitrochlorbenzol, 62,7 g (1,1 mol) Kaliumfluorid, jedoch 40 g (0,08 mol) Polyethylenglykoldimethylether(500) ein und arbeitet wie in Beispiel 1 beschrieben.

| | 4-Nitrochlorbenzol | Lösungsmittel | KF/mol | Katalysator | Zeit (Stunden) | Reaktionstemperatur | Umsatz % | Ausbeute % |
|---|---|---|---|---|---|---|---|---|
| Bsp.1 | 1 mol | ohne | 1.1 | A+B | 20 | 180 | 100 | 88 |
| Vgl.- bsp.1 | 1 mol | ohne | 1.1 | A 0.01 mol | 20 | 180 | 80 | 68 |
| Vgl.- bsp.2 | 1 mol | ohne | 1.1 | B 0.08 mol | 20 | 180 | 25 | <20 |

A=Tetrakis(diethylamino)phosphoniumbromid
B=Polyethylenglykoldimethylether (500).

Herstellung von 2-Nitrofluorbenzol

Beispiel 2

Herstellung von 2-Nitrofluorobenzol durch Umsetzung von 4-Nitrochlorbenzol mittels Tetrakis(diethylamino)phosphoniumbromid und Polyethylenglykoldimethylether(500) als Katalysator.

Man legt in einem 1.5 l Vierhalskolben, der mit Thermometer, Ankerrührer und Rückflußkühler mit Blasenzähler bestückt ist, 157 g (1 mol) 2-Nitrochlorbenzol, 62,7 g (1,1 mol) Kaliumfluorid und 3,99 g (0,01 mol) Tetrakis (diethylamino)-phosphoniumbromid und 40 g (0,08 mol) Polyethylenglykoldimethylether (500) als Katalysator vor. Anschließend erhitzt man unter Rühren auf die vorgegebene Reaktionstemperatur und läßt der vorgegebenen Zeit entsprechend reagieren. Nach Beendigung der Umsetzung läßt man abkühlen, löst das Reaktionsgemisch in Chlorbenzol, filtriert unlösliche Bestandteile (Salze wie KCl, KF) ab und reinigt das Wertprodukt (2-Nitrofluorbenzol) durch fraktionierte Destillation unter reduziertem Druck.

Vergleichsbeispiel 3

Herstellung von 2-Nitrofluorbenzol durch Umsetzung von 2-Nitrochlorbenzol mittels Tetrakis(diethylamino)phosphoniumbromid als Katalysator.

Man setzt 157 g (1 mol) 2-Nitrochlorbenzol, 62,7 g (1,1 mol) Kaliumfluorid, jedoch 3,99 g (0,01 mol) Tetrakis (diethylamino)phosphoniumbromid ein und arbeitet wie in Beispiel 2 beschrieben.

Vergleichsbeispiel 4

Herstellung von 2-Nitrofluorbenzol durch Umsetzung von 2-Nitrochlorbenzol mittels Polyethylenglykoldimethylether (500) als Katalysator.

Man setzt 157 g (1 mol) 2-Nitrochlorbenzol, 62,7 g (1,1 mol) Kaliumfluorid, jedoch 40 g (0,08 mol) Polyethylenglykoldimethylether(500) ein und arbeitet wie in Beispiel 2 beschrieben.

| | 2-Nitrochlorbenzol | Lösungsmittel | KF/mol | Katalysator | Zeit (Stunden) | Reaktionstemperatur | Umsatz % | Ausbeute % |
|---|---|---|---|---|---|---|---|---|
| Bsp.2 | 1 mol | ohne | 1.1 | A+B | 15 | 180 | 99 | 83 |
| Vgl.- bsp.3 | 1 mol | ohne | 1.1 | 0.01 mol A | 15 | 180 | 90 | 68 |
| Vgl.- bsp.4 | 1 mol | ohne | 1.1 | 0.08 mol B | 15 | 180 | 78 | 74 |

A=Tetrakis(diethylamino)phosphoniumbromid

B=Polyethylenglykol(500)dimethylether

Beispiel 3

Herstellung von 2,6-Difluorobenzaldehyd durch Umsetzung von 2,6-Dichlorbenzaldehyd mittels Tetrakis(diethylamino) phosphoniumbromid und Trimethyl(ethoxypolyoxypropyl)- ammoniumchlorid als Katalysator.

Man legt in einem 1,5 l Vierhalskolben, der mit Thermometer, Ankerrührer und Rückflußkühler mit Blasenzähler bestückt ist, 174 g (1 mol) 2,6-Dichlorbenzaldehyd, 114 g (2 mol) Kaliumfluorid und 7,98 g (0,02 mol) Tetrakis (diethylamino)-phosphoniumbromid und 36 g (0,05 mol) Trimethyl(ethoxypolyoxypropyl)-ammoniumchlorid als Katalysator vor. Anschließend erhitzt man unter Rühren auf die vorgegebene Reaktionstemperatur und läßt der vorgegebenen Zeit entsprechend reagieren.
Nach Beendigung der Umsetzung läßt man abkühlen, löst das Reaktionsgemisch in Chlorbenzol, filtriert unlösliche Bestandteile (Salze wie KCl, KF) ab und reinigt das Wertprodukt 2,6-Difluorobenzaldehyd durch fraktionierte Destillation unter reduziertem Druck.

Vergleichsbeispiel 5

Herstellung von 2,6-Difluorobenzaldehyd durch Umsetzung von 2,6-Dichlorbenzaldehyd mittels Tetrakis(diethylamino) phosphoniumbromid als Katalysator.

Man setzt 174 g (1 mol) 2,6-Dichlorbenzaldehyd, 114 g (2 mol) Kaliumfluorid und 7,98 g (0,02 mol) Tetrakis (diethylamino)phosphoniumbromid ein und arbeitet wie in Beispiel 3 beschrieben.

Vergleichsbeispiel 6

Herstellung von 2,6-Difluorobenzaldehyd durch Umsetzung von 2,6-Dichlorbenzaldehyd mittels Trimethyl(ethoxypolyoxypropyl)ammoniumchlorid als Katalysator.

Man setzt 174 g (1 mol) 2,6-Dichlorbenzaldehyd, 114 g (2 mol) Kaliumfluorid und 36 g (0,05 mol) Trimethyl (ethoxypolyoxypropyl)ammoniumchlorid ein und arbeitet wie in Beispiel 3 beschrieben.

| | 2,6-Dichlorbenzaldehyd | Lösungsmittel | KF/mol | Katalysator | Zeit (Stunden) | Reaktionstemperatur | Umsatz % | Ausbeute % |
|---|---|---|---|---|---|---|---|---|
| Bsp.3 | 1 mol | ohne | 2 | A+B | 20 | 165 | 88 | 69 |
| Vgl.- bsp.5 | 1 mol | ohne | 2 | 0.01 mol A | 20 | 165 | 65 | 55 |
| Vgl.- bsp.6 | 1 mol | ohne | 2 | 0.05 mol B | 20 | 165 | 42 | <40 |

A: Tetrakis(diethylamino)phosphonium bromid

B: Trimethyl(ethoxypolyoxypropyl)ammoniumchlorid

Beispiel 4

Herstellung von 3,5-Difluorochlorbenzol durch Umsetzung von 1,3,5-Trichlorbenzol mittels Tetrakis(diethylamino)phosphoniumbromid und Trimethyl(ethoxypolyoxypropyl)ammoniumchlorid als Katalysator.

Man legt in einem 1,5 l Vierhalskolben, der mit Thermometer, Ankerrührer und Rückflußkühler mit Blasenzähler bestückt ist, 180 g (1 mol) 1,3,5-Trichlorbenzol, 114 g (2 mol) Kaliumfluorid und 7,98 g (0,02 mol) Tetrakis (diethylamino)-phosphoniumbromid und 36 g (0,05 mol) Trimethyl(ethoxypolyoxypropyl)-ammoniumchlorid als Katalysator vor. Anschließend erhitzt man unter Rühren auf die vorgegebene Reaktionstemperatur und läßt der vorgegebenen Zeit entsprechend reagieren.
Nach Beendigung der Umsetzung läßt man abkühlen, löst das Reaktionsgemisch in Chlorbenzol, filtriert unlösliche Bestandteile (Salze wie KCl, KF) ab und reinigt das Wertprodukt 3,5-Difluorochlorbenzol durch fraktionierte Destillation.

Vergleichsbeispiel 7

Herstellung von 3,5-Difluorochlorbenzol durch Umsetzung von 1,3,5-Trichlorbenzol mittels Tetrakis(diethylamino)phosphoniumbromid als Katalysator.

Man setzt 180 g (1 mol) 1,3,5-Trichlorbenzol, 114 g (2 mol) Kaliumfluorid und 7,98 g (0,02 mol) Tetrakis (diethylamino)phosphoniumbromid ein und arbeitet wie in Beispiel 4 beschrieben.

Vergleichsbeispiel 8

Herstellung von 3,5-Difluorochlorbenzol durch Umsetzung von 1,3,5-Trichlorbenzol mittels Trimethyl(ethoxypolyoxypropyl)ammoniumchlorid als Katalysator.

Man setzt 180 g (1 mol) 1,3,5-Trichlorbenzol, 114 g (2 mol) Kaliumfluorid und 36 g (0,05 mol) Trimethyl(ethoxypolyoxypropyl)ammoniumchlorid ein und arbeitet wie in Beispiel 4 beschrieben.

| | 1,3,5-Trichlorbenzol | Lösungsmittel | KF/mol | Katalysator | Zeit (Stunden) | Reaktionstemperatur | Umsatz % | Ausbeute % |
|---|---|---|---|---|---|---|---|---|
| Bsp.4 | 1 mol | ohne | 2 | A+B | 24 | 190 | 65 | 61 * |
| Vgl.- bsp.7 | 1 mol | ohne | 2 | 0.01 mol A | 24 | 190 | 40 | 55 ** |
| Vgl.- bsp.8 | 1 mol | ohne | 2 | 0.05 mol B | 24 | 190 | <5 | <5 |

A: Tetrakis(diethylamino)phosphonium bromid
B: Trimethyl(ethoxypolyoxypropyl)ammoniumchlorid
* plus 35% 1,3-Dichlor-5-fluorbenzol
** plus 40% 1,3-Dichlor-5-fluorbenzol

Beispiel 5

Herstellung von 4-Fluorobenzaldehyd durch Umsetzung von 4-Chlorbenzaldehyd mittels Tetrakis(diethylamino)phosphoniumbromid und Tetraphenylphosphoniumbromid als Katalysator.

Man legt in einem 1,5 l Vierhalskolben, der mit Thermometer, Ankerrührer und Rückflußkühler mit Blasenzähler bestückt ist, 140 g (1 mol) 4-Chlorbenzaldehyd, 57 g (1 mol) Kaliumfluorid und 3,99 g (0,01 mol) Tetrakis(diethylamino)-phosphoniumbromid und 4,19 g (0,01 mol) Tetraphenylphosphoniumbromid als Katalysator vor. Anschließend erhitzt man unter Rühren auf die vorgegebene Reaktionstemperatur und läßt der vorgegebenen Zeit entsprechend reagieren. Nach Beendigung der Umsetzung läßt man abkühlen, löst das Reaktionsgemisch in Chlorbenzol, filtriert unlösliche Bestandteile (Salze wie KCl, KF) ab und reinigt das Wertprodukt (4-Fluorobenzaldehyd) durch fraktionierte Destillation unter reduziertem Druck.
Ausbeute 104 g (84%).

Vergleichsbeispiel 9

Herstellung von 4-Fluorobenzaldehyd durch Umsetzung von 4-Chlorbenzaldehyd mittels Tetraphenylphosphoniumbromid als Katalysator.

Man setzt 140 g (1 mol) von 4-Fluorobenzaldehyd, 57 g (1 mol) Kaliumfluorid und 8,4 g (0,02 mol) Tetraphenylphosphoniumbromid ein und arbeitet wie in Beispiel 5 beschrieben.
Ausbeute 32 %.

**Patentansprüche**

1. Katalysator für Halogen-Fluor-Austauschreaktionen an Aromaten, bestehend im wesentlichen aus einer Mischung aus einer oder mehreren Verbindungen der Komponente a) sowie mindestens einer Verbindung der Komponente b), c) und/oder d), wobei die Komponenten

a) ein Amidophosphoniumsalz der Formel (I)

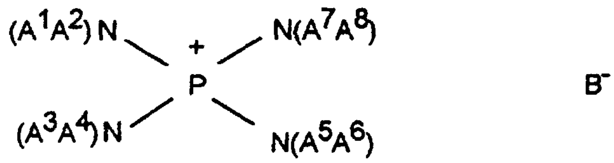

(I)

worin $A^1$, $A^2$, $A^3$, $A^4$, $A^5$, $A^6$, $A^7$, $A^8$ unabhängig voneinander, gleich oder verschieden sind und für ein geradkettiges oder verzweigtes Alkyl oder Alkenyl mit 1 bis 12 Kohlenstoffatomen, Cycloalkyl mit 4 bis 8 Kohlenstoffatomen, für ein Aryl mit 6 bis 12 Kohlenstoffatomen, ein Aralkyl mit 7 bis 12 Kohlenstoffatomen stehen, oder $A^1 A^2$, $A^3A^4$, $A^5A^6$, $A^7A^8$ unabhängig voneinander, gleich oder verschieden sind und direkt oder über O oder N-$A^9$ miteinander zu einem Ring mit 3 bis 7 Ringgliedem verbunden sind, $A^9$ für ein Alkyl mit 1 bis 4 Kohlenstoffatomen steht und $B^-$ einen einwertigen Säurerest oder das Äquivalent eines mehrwertigen Säurerests bedeutet,

b) eine quartäre Ammoniumverbindung der Formel (II)

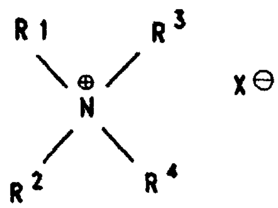

(II)

worin

$R^1$, $R^2$, $R^3$ und $R^4$ gleich oder verschieden sind und einen linearen oder verzweigten Alkoxypolyoxyalkyl-Rest der Formel $-(C_mH_{2m}O)_pR^5$ bedeuten, worin $R^5$ für einen linearen oder verzweigten Alkylrest mit 1 bis 16 Kohlenstoffatomen oder für $C_1$-$C_4$-Alkyl-aryl steht, m eine ganze Zahl von 1 bis 10 und p eine Zahl von 1 bis 15 bedeuten; oder einen linearen oder verzweigten Alkylrest mit 1 bis 30 Kohlenstoffatomen; oder einen unsubstituierten Phenyl- oder Naphthylrest; oder einen substituierten Phenyl- oder Naphthylrest, wobei die Substituenten die Bedeutung Halogen, $C_1$-$C_4$-Alkyl, $C_1$-$C_4$-Alkoxy, Nitro, $CF_3$ oder Cyano haben; und

$X^{\ominus}$ ein Anion ist;

und

c) eine quartäre Phosphoniumverbindung der Formel (III)

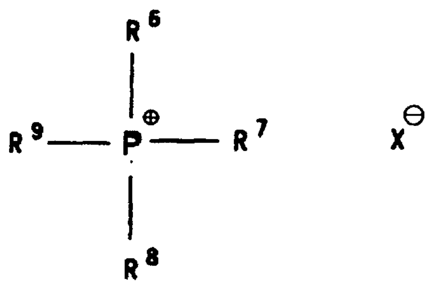

(III) ,

worin

$R^6$, $R^7$, $R^8$ und $R^9$ gleich oder verschieden sind und einen linearen oder verzweigten Alkylrest mit 1 bis 22 Kohlenstoffatomen; oder einen unsubstituierten oder substituierten Arylrest oder einen $C_1$-$C_4$-Alkyl-arylrest, wobei Aryl die Bedeutung Phenyl oder Naphthyl hat und die besagten Substituenten Halogen, $C_1$-$C_4$-Alkyl, $C_1$-$C_4$-Alkoxy, Nitro oder Cyano bedeuten;

$X^{\ominus}$ wie vorstehend definiert ist; und

d) ein Kronenether oder ein Polyether der Formel (IV)

$$R^{10}\text{-}(O\text{-}C_xH_{2x})_r\text{-}OR^{11} \quad \text{(IV)},$$

worin

$R^{10}$ und $R^{11}$ gleich oder verschieden sind und einen linearen oder verzweigten Alkylrest mit 1 bis 16 Kohlenstoffatomen bedeuten;

x eine ganze Zahl von 2 bis 6 und

r eine Zahl von 0 bis 20 ist,

bedeuten.

**2.** Katalysator nach Anspruch 1, bestehend aus Komponente a) und b), wobei mindestens einer der Reste $R^1$, $R^2$, $R^3$ und $R^4$ einen linearen oder verzweigten Alkoxypolyoxyalkyl-Rest der Formel $-(C_mH_{2m}O)_pR^5$- bedeutet, oder bestehend aus Komponente a) und d).

**3.** Katalysator nach Anspruch 1 oder 2, **dadurch gekennzeichnet, daß** die Komponente a) 5 bis 95 Gew.-%, vorzugsweise 10 bis 80 Gew.-%, des gesamten Katalysators ausmacht.

**4.** Katalysator nach mindestens einem der Ansprüche 1 bis 3, **dadurch gekennzeichnet, daß** die Komponente a)

eine oder mehrere der Verbindungen

Tetrakis(dimethylamino)phosphoniumchlorid,
Tetrakis(diethylamino)phosphoniumchlorid,
Tetrakis(dimethylamino)phosphoniumbromid,
Tetrakis(diethylamino)phosphoniumbromid,
Tetrakis(dipropylamino)phosphoniumchlorid oder -bromid,
Tris(diethylamino)(dimethylamino)phosphoniumchlorid oder -bromid,
Tetrakis(dibutylamino)phosphoniumchlorid oder -bromid,
Tris(dimethylamino)(diethylamino)phosphoniumchlorid oder -bromid,
Tris(dimethylamino)(cyclopentylamino)phosphoniumchlorid oder -bromid,
Tris(dimethylamino)(dipropylamino)phosphoniumchlorid oder -bromid,
Tris(dimethylamino)(dibutylamino)phosphoniumchlorid oder -bromid,
Tris(dimethylamino)(cyclohexylamino)phosphoniumchlorid oder -bromid,
Tris(dimethylamino)(diallylamino)phosphoniumchlorid oder -bromid,
Tris(dimethylamino)(dihexylamino)phosphoniumchlorid oder-bromid,
Tris(diethylamino)(dihexylamino)phosphoniumchlorid oder -bromid,
Tris(dimethylamino)(diheptylamino)phosphoniumchlorid oder -bromid,
Tris(diethylamino)(diheptylamino)phosphonium chlorid oder -bromid,
Tetrakis(pyrrolidino)phosphoniumchlorid oder -bromid,
Tetrakis(piperidino)phosphoniumchlorid oder -bromid,
Tetrakis(morpholino)phosphoniumchlorid oder -bromid,
Tris(piperidino)(diallylamino)phosphoniumchlorid oder -bromid,
Tris(pyrrolidino)(ethylmethylamino)phosphoniumchlorid oder -bromid,
Tris(pyrrolidino)(diethylamino)phosphoniumchlorid oder -bromid ist.

**5.** Katalysator nach mindestens einem der Ansprüche 1 bis 4, **dadurch gekennzeichnet, daß** die Komponente b) eine oder mehrere der Verbindungen Dimethyl-di(ethoxypolyoxypropyl)-ammoniumchlorid, Dimethyldi(ethoxypolyoxypropylmethylether)-ammoniumchlorid, Dimethyl(ethoxypolyoxypropyl)-(ethoxypolyoxypropylmethylether)-ammoniumchlorid, Dimethyl-di(ethoxypolyoxyethyl)-ammoniumchlorid, Dimethyldi(ethoxypolyoxyethylmethylether)-ammoniumchlorid, Dimethyl(ethoxypolyoxyethyl)-(ethoxypolyoxyethylmethylether)-ammoniumchlorid, jeweils mit einer mittleren Kettenlänge p von 3, oder Trimethyl-(ethoxypolyoxypropyl)-ammoniumchlorid und Trimethyl-(ethoxypolyoxypropylmethylether)-ammoniumchlorid, jeweils mit einer mittleren Kettenlänge p von 8 ist.

**6.** Katalysator nach mindestens einem der Ansprüche 1 bis 5, **dadurch gekennzeichnet, daß** die Komponente c) eine oder mehrere der Verbindungen Hexadecyltributylphosphoniumbromid, Stearyltributylphosphoniumbromid, Tetrabutylphosphoniumchlorid, Tetrabutylphosphoniumbromid, Tetraoctylphosphoniumbromid, Tetraphenylphosphoniumchlorid oder -bromid ist.

**7.** Katalysator nach mindestens einem der Ansprüche 1 bis 5, **dadurch gekennzeichnet, daß** die Komponente d) ein Gemisch von Polyethylenglykoldimethylethern der Kettenlängen r von 6 bis 17 ist.

**8.** Katalysator nach Anspruch 1, bestehend aus Tetrakis(diethylamino)-phosphoniumbromid oder -chlorid und einem Polyethylenglykoldimethylether, oder bestehend aus Tetrakis(diethylamino)phosphoniumbromid oder -chlorid und Trimethyl(ethoxypolyoxypropyl)ammoniumchlorid.

**9.** Verwendung des Katalysators gemäß einem oder mehreren der Ansprüche 1 bis 8 für Halogen-Fluor-Austauschreaktionen an Aromaten, **dadurch gekennzeichnet, daß** man eine Verbindung, die gegen Fluor austauschbares Halogen enthält, mit einem Fluorid oder einem Gemisch von Fluoriden der allgemeinen Formel (V)

$$MeF \qquad (V),$$

worin Me für ein stöchiometrisches Equivalent eines Erdalkalimetallions, ein Alkalimetallion oder ein Tetraalkylammonium-Ion steht, in Gegenwart des besagten Katalysators, in Gegenwart oder Abwesenheit eines Lösungsmittels bei einer Temperatur von 40 bis 260°C umsetzt.

**10.** Verwendung nach Anspruch 9, **dadurch gekennzeichnet, daß** der Katalysator in einer Menge von 0,5 bis 35

Gew.-%, bevorzugt 3 bis 25 Gew.-%, bezogen auf die Verbindung, die gegen Fluor austauschbares Halogen enthält, eingesetzt wird.

**11.** Verwendung nach Anspruch 9 oder 10, **dadurch gekennzeichnet, daß** man als Verbindung, die gegen Fluor austauschbares Halogen enthält, eine am Kern einen gegen Fluor austauschbaren Chlor- oder Bromsubstituenten besitzende aromatische Verbindung mit 0 bis 3 Stickstoffatomen im Kern, die gegebenenfalls wenigstens einen weiteren, die nucleophile Substitution der aromatischen Verbindungen begünstigenden Substituenten aufweist, einsetzt.

**12.** Verwendung nach einem oder mehreren der Ansprüche 9 bis 11, **dadurch gekennzeichnet, daß** man eine am Kern einen gegen Fluor austauschbaren Chloroder Bromsubstituenten besitzende aromatische Verbindung, die wenigstens einen weiteren Substituenten aus der Reihe F, Cl, Br, J, $NO_2$, $CF_3$, CN, CHO, COF, COCl, $SO_2F$, $SO_2Cl$, $OCF_3$, $SCF_3$, $SOCF_3$, $SO_2CF_3$, COOR, CONRR', $SO_2R$, COR oder OR oder einen Rest -CO-O-CO-, -CO-NR-CO-, der zwei ortho-Stellungen verknüpft, aufweist, wobei R und R' jeweils unabhängig voneinander, gleich oder verschieden sind, und für H, ein geradkettiges oder verzweigtes Alkyl mit 1 bis 6 Kohlenstoffatomen, ein Aryl mit 6 bis 12 Kohlenstoffatomen oder Aralkyl mit 7 bis 12 Kohlenstoffatomen stehen und die Alkyle und Aralkyle gegebenenfalls einfach bis dreifach halogensubstituiert sind, einsetzt.

**13.** Verwendung nach einem oder mehreren der Ansprüche 9 bis 12, **dadurch gekennzeichnet, daß** man eine am Kern einen gegen Fluor austauschbaren Chloroder Bromsubstituenten besitzende aromatische Verbindung, die wenigstens ein gegen Fluor austauschbares Chlor oder Brom als weiteren Substituenten und gegebenenfalls wenigstens einen weiteren Substituenten aus der Reihe F, $NO_2$, $CF_3$, CN, CHO, COF, COCl, $SO_2F$, $SO_2Cl$, $OCF_3$, $SCF_3$, $SO_2CF_3$, COOR, CONRR', $SO_2R$, COR, OR, -CO-O-CO- oder -CO-NR-CO- besitzt, einsetzt.

**14.** Verwendung nach einem oder mehreren der Ansprüche 9 bis 13, **dadurch gekennzeichnet, daß** man eine Verbindung der allgemeinen Formel (VI)

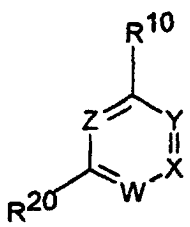

(VI)

worin W für N oder C-$R^{30}$, X für N oder C-$R^{40}$, Y für N oder C-$R^{50}$, Z für N oder C-$R^{60}$ steht, W, X und Y nicht zugleich N sind, $R^{10}$, $R^{20}$, $R^{30}$, $R^{40}$, $R^{50}$, $R^{60}$ gleich oder verschieden sind und H, F, Cl, Br, J, $NO_2$, NO, $CF_3$, CN, CHO, COF, COCl, $SO_2F$, $SO_2Cl$, $OCF_3$, $SCF_3$, $SO_2CF_3$, COOR, CONRR', $SO_2R$, COR, OR, einen Rest -CO-O-CO, -CO-NR-CO- oder -CR"=CR"-CR"=CR"-, der zwei ortho-Stellungen verknüpft, bedeuten, R und R' die vorstehende Bedeutung besitzen und R" unabhängig voneinander, gleich oder verschieden sind und dieselbe Bedeutung wie $R^{10}$ bis $R^{60}$ haben, und wenigstens einer der Reste $R^{10}$ bis $R^{60}$ Chlor oder Brom ist, einsetzt.

**15.** Verwendung nach einem oder mehreren der Ansprüche 9 bis 14, **dadurch gekennzeichnet, daß** man eine Verbindung der Formel (VI), worin nur einer der Reste $R^{10}$ bis $R^{60}$ Chlor oder Brom ist, keiner der Reste W, X, Y, Z für ein Stickstoffatom steht und wenigstens einer der verbleibenden Reste aus der Gruppe $R^{10}$ bis $R^{60}$ $NO_2$, $CF_3$, CN, CHO, COF, COCl, $SO_2F$, $SO_2Cl$, $OCF_3$, $SCF_3$, $SO_2CF_3$, COOR, COONRR', $SO_2R$, COR, OR, -CO-O-CO-, -CO-NR-CO- oder -CR"=CR"-CR"=CR"- ist, einsetzt.

**16.** Verwendung nach einem oder mehreren der Ansprüche 9 bis 14, **dadurch gekennzeichnet, daß** man eine Verbindung der Formel (VI), worin zwei oder mehrere der Reste $R^{10}$ bis $R^{60}$ Chlor oder Brom sind, die Reste W, X, Y, Z für 0 bis 3 Stickstoffatome stehen und die verbleibenden Reste aus der Gruppe $R^{10}$ bis $R^{60}$ alle Wasserstoff sein können, einsetzt.

**17.** Verwendung nach einem oder mehreren der Ansprüche 9 bis 16, **dadurch gekennzeichnet, daß** man als Fluorid der Formel (V) Calciumfluorid, Ammoniumfluorid, Lithiumfluorid, Natriumfluorid, Kaliumfluorid, Rubidiumfluorid, Cäsiumfluorid oder ein Gemisch derselben einsetzt.

**18.** Verwendung nach einem oder mehreren der Ansprüche 9 bis 17, **dadurch gekennzeichnet, daß** man das Fluorid der Formel (V): Äquivalent auszutauschendes Halogen im Verhältnis (0,5 bis 10):(1), insbesondere (0,8 bis 5):(1), bevorzugt (1 bis 2):(1) einsetzt.

## Claims

**1.** A catalyst for halogen-fluorine exchange reactions on aromatics, consisting essentially of a mixture of one or more compounds of the component a) plus at least one compound of the components b), c) and/or d), wherein the component

a) is an amidophosphonium salt of the formula (I)

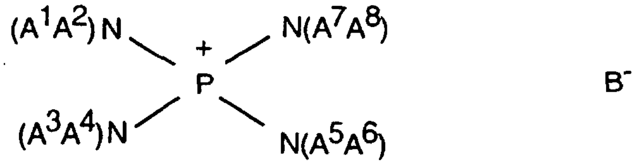

(I)

where $A^1$, $A^2$, $A^3$, $A^4$, $A^5$, $A^6$, $A^7$, $A^8$ are, independently of one another, identical or different and are each a straight-chain or branched alkyl or alkenyl having from 1 to 12 carbon atoms, cycloalkyl having from 4 to 8 carbon atoms, an aryl having from 6 to 12 carbon atoms, an aralkyl having from 7 to 12 carbon atoms, or $A^1A^2$, $A^3A^4$, $A^5A^6$, $A^7A^8$ are, independently of one another, identical or different and are in each case connected to one another either directly or via O or N-$A^9$ to form a ring having from 3 to 7 ring atoms, $A^9$ is an alkyl having from 1 to 4 carbon atoms and $B^-$ is a monovalent acid anion or the equivalent of a polyvalent acid anion,

b) is a quaternary ammonium compound of the formula (II)

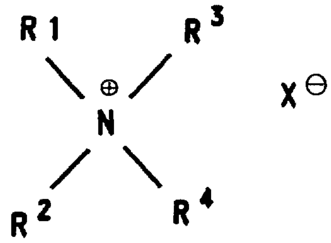

(II)

where

| | |
|---|---|
| $R^1$, $R^2$, $R^3$ and $R^4$ | are identical or different and are each a linear or branched alkoxypolyoxyalkyl radical of the formula $-(C_mH_{2m}O)_pR^5$, where $R^5$ is a linear or branched alkyl radical having from 1 to 16 carbon atoms or $C_1$-$C_4$-alkylaryl, m is an integer from 1 to 10, and p is a number from 1 to 15; or a linear or branched alkyl radical having from 1 to 30 carbon atoms; or an unsubstituted phenyl or naphthyl radical; or a substituted phenyl or naphthyl radical, where the substituents are halogen, $C_1$-$C_4$-alkyl, $C_1$-$C_4$-alkoxy, nitro, $CF_3$ or cyano; and |
| $X^\ominus$ | is an anion; |

and

c) is a quaternary phosphonium compound of the formula (III)

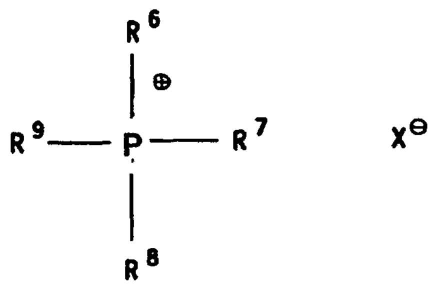

(III),

where

| | |
|---|---|
| $R^6$, $R^7$, $R^8$ and $R^9$ | are identical or different and are each a linear or branched alkyl radical having from 1 to 22 carbon atoms; or an unsubstituted or substituted aryl radical or a $C_1$-$C_4$-alkylaryl radical, where aryl is phenyl or naphthyl and said substituents are halogen, $C_1$-$C_4$-alkyl, $C_1$-$C_4$-alkoxy, nitro or cyano; |
| $X^{\ominus}$ | is as defined above; and |

d) is a crown ether or a polyether of the formula (IV)

$$R^{10}\text{-}(O\text{-}C_xH_{2x})_r\text{-}OR^{11} \qquad \text{(IV)},$$

where

| | |
|---|---|
| $R^{10}$ and $R^{11}$ | are identical or different and are each a linear or branched alkyl radical having from 1 to 16 carbon atoms; |
| x | is an integer from 2 to 6 and |
| r | is a number from 0 to 20. |

**2.** A catalyst as claimed in claim 1 consisting of component a) and b), where at least one of the radicals $R^1$, $R^2$, $R^3$ and $R^4$ is a linear or branched alkoxypolyoxyalkyl radical of the formula $-(C_mH_{2m}O)_pR^5$, or consisting of component a) and d).

**3.** A catalyst as claimed in claim 1 or 2, wherein the component a) makes up from 5 to 95% by weight, preferably from 10 to 80% by weight, of the total catalyst.

**4.** A catalyst as claimed in at least one of claims 1 to 3, wherein the component a) is one or more of the compounds

tetrakis(dimethylamino)phosphonium chloride,
tetrakis(diethylamino)phosphonium chloride,
tetrakis(dimethylamino)phosphonium bromide,
tetrakis(diethylamino)phosphonium bromide,
tetrakis(dipropylamino)phosphonium chloride or bromide,
tris(diethylamino)(dimethylamino)phosphonium chloride or bromide,
tetrakis(dibutylamino)phosphonium chloride or bromide,
tris(dimethylamino)(diethylamino)phosphonium chloride or bromide,
tris(dimethylamino)(cyclopentylamino)phosphonium chloride or bromide,
tris(dimethylamino)(dipropylamino)phosphonium chloride or bromide,
tris(dimethylamino)(dibutylamino)phosphonium chloride or bromide,
tris(dimethylamino)(cyclohexylamino)phosphonium chloride or bromide,
tris(dimethylamino)(diallylamino)phosphonium chloride or bromide,
tris(dimethylamino)(dihexylamino)phosphonium chloride or bromide,
tris(diethylamino)(dihexylamino)phosphonium chloride or bromide,
tris(dimethylamino)(diheptylamino)phosphonium chloride or bromide,

tris(diethylamino)(diheptylamino)phosphonium chloride or bromide,
tetrakis(pyrrolidino)phosphonium chloride or bromide,
tetrakis(piperidino)phosphonium chloride or bromide,
tetrakis(morpholino)phosphonium chloride or bromide,
tris(piperidino)(diallylamino)phosphonium chloride or bromide,
tris(pyrrolidino)(ethylmethylamino)phosphonium chloride or bromide,
tris(pyrrolidino)(diethylamino)phosphonium chloride or bromide.

**5.** A catalyst as claimed in at least one of claims 1 to 4, wherein the component b) is one or more of the compounds dimethyldi(ethoxypolyoxypropyl)ammonium chloride, dimethyldi(ethoxypolyoxypropyl methyl ether)ammonium chloride, dimethyl(ethoxypolyoxypropyl)(ethoxypolyoxypropyl methyl ether)ammonium chloride, dimethyldi(ethoxypolyoxyethyl)ammonium chloride, dimethyldi(ethoxypolyoxyethyl methyl ether)ammonium chloride, dimethyl (ethoxypolyoxyethyl)(ethoxypolyoxyethyl methyl ether)ammonium chloride, in each case having a mean chain length p of 3, or trimethyl(ethoxypolyoxypropyl)ammonium chloride and trimethyl-(ethoxypolyoxypropyl methyl ether)ammonium chloride, in each case having a mean chain length p of 8.

**6.** A catalyst as claimed in at least one of claims 1 to 5, wherein the component c) is one or more of the compounds hexadecyltributylphosphonium bromide, stearyltributylphosphonium bromide, tetrabutylphosphonium chloride, tetrabutylphosphonium bromide, tetraoctylphosphonium bromide, tetraphenylphosphonium chloride or bromide.

**7.** A catalyst as claimed in at least one of claims 1 to 5, wherein the component d) is a mixture of polyethylene glycol dimethyl ethers having chain lengths r of from 6 to 17.

**8.** A catalyst as claimed in claim 1 consisting of tetrakis(diethylamino)-phosphonium bromide or chloride and a polyethylene glycol dimethyl ether or consisting of tetrakis(diethylamino)phosphonium bromide or chloride and trimethyl(ethoxypolyoxypropyl)ammonium chloride.

**9.** The use of a catalyst as claimed in one or more of claims 1 to 8 for halogen-fluorine exchange reactions on aromatics, wherein a compound containing halogen which can be replaced by fluorine is reacted with a fluoride or a mixture of fluorides of the formula (V)

$$MeF \qquad (V),$$

where Me is one stoichiometric equivalent of an alkaline earth metal ion, an alkali metal ion or a tetraalkylammonium ion, in the presence of said catalyst, in the presence or absence of a solvent, at a temperature of from 40 to 260°C.

**10.** The use as claimed in claim 9, wherein the catalyst is used in an amount of from 0.5 to 35% by weight, preferably from 3 to 25% by weight, based on the compound containing the halogen which can be replaced by fluorine.

**11.** The use as claimed in claim 9 or 10, wherein the compound containing halogen which can be replaced by fluorine is an aromatic compound bearing, on the ring, a chloro or bromo substituent which can be replaced by fluorine and having from 0 to 3 nitrogen atoms in the ring, which compound may bear at least one further substituent which favors the nucleophilic substitution of the aromatic compounds.

**12.** The use as claimed in one or more of claims 9 to 11, wherein use is made of an aromatic compound bearing, on the ring, a chloro or bromo substituent which can be replaced by fluorine, which compound bears at least one further substituent selected from the group consisting of F, Cl, Br, I, $NO_2$, $CF_3$, CN, CHO, COF, COCl, $SO_2F$, $SO_2Cl$, $OCF_3$, $SCF_3$, $SOCF_3$, $SO_2CF_3$, COOR, CONRR', $SO_2R$, COR or OR or a radical -CO-O-CO-, -CO-NR-CO- which links two ortho positions, where R and R' are, independently of one another, identical or different and are each H, a straight-chain or branched alkyl having from 1 to 6 carbon atoms, an aryl having from 6 to 12 carbon atoms or aralkyl having from 7 to 12 carbon atoms, and the alkyls and aralkyls may be singly to triply halogen-substituted.

**13.** The use as claimed in one or more of claims 9 to 12, wherein use is made of an aromatic compound bearing, on the ring, a chloro or bromo substituent which can be replaced by fluorine, which compound bears at least one chlorine or bromine, which can be replaced by fluorine as further substituents and, if desired, at least one further substituent selected from the group consisting of F, $NO_2$, $CF_3$, CN, CHO, COF, COCl, $SO_2F$, $SO_2Cl$, $OCF_3$, $SCF_3$, $SO_2CF_3$, COOR, CONRR', $SO_2R$, COR, OR, -CO-O-CO- or -CO-NR-CO-.

**14.** The use as claimed in one or more of claims 9 to 13, wherein use is made of a compound of the formula (VI)

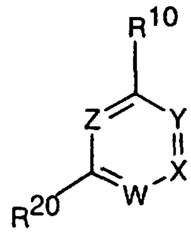

(VI)

where W is N or C-$R^{30}$, X is N or C-$R^{40}$, Y is N or C-$R^{50}$, Z is N or C-$R^{60}$, W, X and Y are not simultaneously N, $R^{10}$, $R^{20}$, $R^{30}$, $R^{40}$, $R^{50}$, $R^{60}$ are identical or different and are H, F, Cl, Br, I, $NO_2$, NO, $CF_3$, CN, CHO, COF, COCl, $SO_2F$, $SO_2Cl$, $OCF_3$, $SCF_3$, $SO_2CF_3$, COOR, CONRR', $SO_2R$, COR, OR or a radical -CO-O-CO, -CO-NR-CO- or -CR"=CR"-CR"=CR"- which links two ortho positions, R and R' are as defined above and R" are, independently of one another, identical or different and have the meanings given for $R^{10}$ to $R^{60}$, and at least one of the radicals $R^{10}$ to $R^{60}$ is chlorine or bromine.

**15.** The use as claimed in one or more of claims 9 to 14, wherein use is made of a compound of the formula (VI) in which only one of the radicals $R^{10}$ to $R^{60}$ is chlorine or bromine, none of the radicals W, X, Y, Z is a nitrogen atom and at least one of the remaining radicals from the group $R^{10}$ to $R^{60}$ is $NO_2$, $CF_3$, CN, CHO, COF, COCl, $SO_2F$, $SO_2Cl$, $OCF_3$, $SCF_3$, $SO_2CF_3$, COOR, COONRR', $SO_2R$, COR, OR, -CO-O-CO-, -CO-NR-CO- or -CR"=CR"-CR"=CR"-.

**16.** The use as claimed in one or more of claims 9 to 14, wherein use is made of a compound of the formula (VI) in which two or more of the radicals $R^{10}$ to $R^{60}$ are chlorine or bromine, the radicals W, X, Y, Z are from 0 to 3 nitrogen atoms and the remaining radicals from the group $R^{10}$ to $R^{60}$ can all be hydrogen.

**17.** The use as claimed in one or more of claims 9 to 16, wherein the fluoride of the formula (V) which is used is calcium fluoride, ammonium fluoride, lithium fluoride, sodium fluoride, potassium fluoride, rubidium fluoride, cesium fluoride or a mixture thereof.

**18.** The use as claimed in one or more of claims 9 to 17, wherein the ratio of fluoride of the formula (V): equivalents of halogen to be replaced is (0.5 - 10):(1), in particular (0.8 - 5): (1), preferably (1 - 2):(1).

## Revendications

**1.** Catalyseur de réactions d'échange halogène-fluor sur des composés aromatiques, constitué essentiellement par un mélange d'un ou plusieurs composés du composant a) ainsi qu'au moins d'un composé du composant b), c) et/ou d), les composants étant

a) un sel d'amidophosphonium de formule (I)

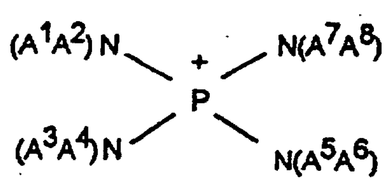

$B^-$ (I)

où $A^1$, $A^2$, $A^3$, $A^4$, $A^5$, $A^6$, $A^7$, $A^8$ sont indépendamment les uns des autres identiques ou différents, et représentent un groupe alkyle ou alcényle linéaire ou ramifié présentant 1 à 12 atomes de carbone, un groupe cycloalkyle présentant 4 à 8 atomes de carbone, un groupe aryle présentant 6 à 12 atomes de carbone, un groupe aralkyle présentant 7 à 12 atomes de carbone, où $A^1A^2$, $A^3A^4$, $A^5A^6$, $A^7A^8$ sont indépendamment les

uns des autres identiques ou différents, et sont reliés les uns aux autres directement ou par l'intermédiaire d'un atome de O ou d'un groupe N-$A^9$ en un cycle de 3 à 7 chaînons, $A^9$ représente un groupe alkyle présentant 1 à 4 atomes de carbone et $B^-$ représente un reste acide monovalent ou l'équivalent d'un reste acide multivalent,

b) un composé d'ammonium quaternaire de formule (II)

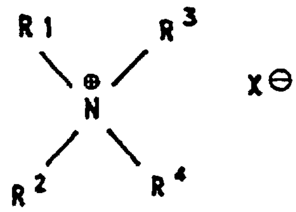

(II)

où

$R^1$, $R^2$, $R^3$ et $R^4$ sont identiques ou différents et représentent un reste alkoxypolyoxyalkyle linéaire ou ramifié de formule $-(C_mH_{2m}O)_pR^5$, où $R^5$ représente un reste alkyle linéaire ou ramifié présentant 1 à 16 atomes de carbone ou un reste (alkyl en $C_1$-$C_4$)-aryle, m est un entier de 1 à 10 et p va de 1 à 15, ou un reste alkyle linéaire ou ramifié présentant 1 à 30 atomes de carbone ou un reste phényle ou naphtyle non substitué ; ou un reste phényle ou naphtyle substitué, les substituants représentant des atomes d'halogènes, des groupes alkyle en $C_1$-$C_4$, alkoxy en $C_1$-$C_4$, nitro, $CF_3$ ou cyano ; et

$X^\ominus$ représente un anion ;

et

c) un composé de phosphonium quaternaire de formule (III)

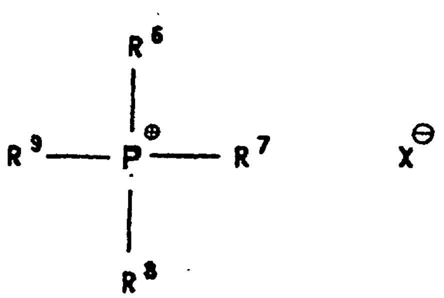

(III),

où

$R^6$, $R^7$, $R^8$ et $R^9$ sont identiques ou différents et représentent un reste alkyle linéaire ou ramifié présentant 1 à 22 atomes de carbone ; ou un reste aryle non substitué ou substitué ou un reste (alkyl en $C_1$-$C_4$)-aryle, le reste aryle ayant la signification de phényle ou naphtyle et lesdits substituants représentent des groupes halogène, alkyle en $C_1$-$C_4$, alkoxy en $C_1$-$C_4$, nitro ou cyano ;

$X^\ominus$ est défini comme ci-dessus, et

d) un éther couronne ou un polyéther de formule (IV)

$$R^{10}\text{-}(O\text{-}C_xH_{2x})_r\text{-}OR^{11} \qquad \text{(IV)},$$

où

$R^{10}$ et $R^{11}$ sont identiques ou différents et représentent un reste alkyle linéaire ou ramifié présentant 1 à

16 atomes de carbone ;
x est un entier de 2 à 6 et
r va de 0 à 20.

**2.** Catalyseur selon la revendication 1, constitué par des composants a) et b), où au moins un des restes $R^1$, $R^2$, $R^3$ et $R^4$ représente un reste alkoxypolyoxyalkyle linéaire ou ramifié de formule $-(C_mH_{2m}O)_pR^5-$ ou constitué par des composants a) et d).

**3.** Catalyseur selon la revendication 1 ou 2, **caractérisé en ce que** le composant a) est présent dans une quantité de 5 à 95 % en poids, de préférence de 10 à 80 % en poids, par rapport à la totalité du catalyseur.

**4.** Catalyseur selon au moins l'une des revendications 1 à 3, **caractérisé en ce que** le composant a) est un ou plusieurs des composés

chlorure de tétrakis(diméthylamino)phosphonium,
chlorure de tétrakis(diéthylamino)phosphonium,
bromure de tétrakis(diméthylamino)phosphonium,
bromure de tétrakis(diéthylamino)phosphonium,
chlorure ou bromure de tétrakis(dipropylamino)-phosphonium,
chlorure ou bromure de tris(diéthylamino)-(diméthylamino)phosphonium
chlorure ou bromure de tétrakis(dibutylamino)-phosphonium,
chlorure ou bromure de tris(diméthylamino)-(diéthylamino)phosphonium,
chlorure ou bromure de tris(diméthylamino)-(cyclopentylamino)phosphonium,
chlorure ou bromure de tris(diméthylamino)-(dipropylamino) phosphonium,
chlorure ou bromure de tris(diméthylamino)-(dibutylamino)phosphonium,
chlorure ou bromure de tris(diméthylamino)-(cyclohexylamino)phosphonium,
chlorure ou bromure de tris(diméthylamino)-(diallylamino)phosphonium,
chlorure ou bromure de tris(diméthylamino)-(dihexylamino)phosphonium,
chlorure ou bromure de tris(diéthylamino)-(dihexylamino)phosphonium,
chlorure ou bromure de tris(diméthylamino)-(diheptylamino)phosphonium,
chlorure ou bromure de tris(diéthylamino)-(diheptylamino)phosphonium,
chlorure ou bromure de tétrakis(pyrrolidino)-phosphonium,
chlorure ou bromure de tétrakis(pipéridino)-phosphonium,
chlorure ou bromure de tétrakis(morpholino)-phosphonium,
chlorure ou bromure de tris(pipéridino)(diallylamino)-phosphonium,
chlorure ou bromure de tris(pyrrolidino)(éthylméthylamino)phosphonium,
chlorure ou bromure de tris(pyrrolidino)(diéthylamino)phosphonium.

**5.** Catalyseur selon au moins l'une des revendications 1 à 4, **caractérisé en ce que** le composant b) est un ou plusieurs des composés chlorure de diméthyl-di(éthoxypolyoxypropyl)ammonium, chlorure de diméthyl-di(éthoxypolyoxypropyl-méthyléther)ammonium, chlorure de diméthyl(éthoxypolyoxypropyl)(éthoxypolyoxypropylméthyléther)ammonium chlorure de diméthyl-di(éthoxypolyoxyéthyl)-ammonium, chlorure de diméthyl-di(éthoxypolyoxyéthylméthyléther)ammonium, chlorure de diméthyl(éthoxypolyoxyéthyl)(éthoxypolyoxyéthylméthyléther)ammonium, chacun ayant une longueur moyenne de chaîne p de 3 ou chlorure de triméthyl(éthoxypolyoxypropyl)ammonium et chlorure de triméthyl-(éthoxypolyoxypropylméthyléther)ammonium, chacun ayant une longueur moyenne de chaîne p de 8.

**6.** Catalyseur selon au moins l'une des revendications 1 à 5, **caractérisé en ce que** le composant c) est un ou plusieurs des composés le bromure d'hexadécyltributylphosphonium, le bromure de stéryltributylphosphonium, le chlorure de tétrabutylphosphonium, le bromure de tétrabutylphosphonium, le chlorure de tétraoctylphosphonium, le chlorure ou le bromure de tétraphénylphosphonium.

**7.** Catalyseur selon au moins l'une des revendications 1 à 5, **caractérisé en ce que** le composant d) est un mélange d'éthers diméthyliques du polyéthylèneglycol ayant une longueur de chaîne r de 6 à 17.

**8.** Catalyseur selon la revendication 1, constitué par le chlorure ou le bromure de tétrakis(diéthylamino)phosphonium et un éther diméthylique du polyéthylèneglycol, ou constitué par le bromure ou le chlorure de tétrakis(diéthylamino)-phosphonium et le chlorure de triméthyl(éthoxypolyoxypropyl)ammonium.

**9.** Utilisation du catalyseur selon une ou plusieurs des revendications 1 à 8 pour des réactions d'échange halogène-fluor sur des composés aromatiques, **caractérisé en ce qu'**on fait réagir un composé qui contient un atome d'halogène échangeable contre le fluor, sur un fluorure ou un mélange de fluorures de formule générale (V)

$$MeF \quad (V),$$

où Me représente un équivalent stoechiométrique d'un ion métal alcalino-terreux, d'un ion métal alcalin ou d'un ion tétraalkylammonium, en présence dudit catalyseur, en présence ou en l'absence d'un solvant à une température de 40 à 260°C.

**10.** Utilisation selon la revendication 9, **caractérisée en ce qu'**on utilise le catalyseur dans une quantité de 0,5 à 35 % en poids, de préférence de 3 à 25 % en poids, par rapport au composé qui contient l'atome d'halogène échangeable contre l'atome de fluor.

**11.** Utilisation selon la revendication 9 ou 10, **caractérisée en qu'**on utilise, en tant que composé contenant un atome d'halogène échangeable contre un atome de fluor, un composé aromatique présentant 0 à 3 atomes d'azote dans le cycle, portant sur le cycle un substituant chloro ou bromo échangeable contre un atome de fluor, lequel composé éventuellement présente au moins un autre substituant favorisant la substitution nucléophile des composés aromatiques.

**12.** Utilisation selon une ou plusieurs des revendications 9 à 11, **caractérisée en ce qu'**on utilise un composé aromatique qui porte sur le noyau un substituant chloro ou bromo échangeable contre un atome de fluor qui présente au moins un autre substituant pris dans le groupe comprenant F, Cl, Br, J, $NO_2$, $CF_3$, CN, CHO, COF, COCl, $SO_2F$, $SO_2Cl$, $OCF_3$, $SCF_3$, $SOCF_3$, $SO_2CF_3$, COOR, CONRR', $SO_2R$, COR ou OR ou un reste -CO-O-CO-, -CO-NR-CO-, qui relie deux positions ortho, R et R' étant indépendamment l'un de l'autre identiques ou différents et représentant chacun un atome d'hydrogène, un groupe alkyle linéaire ou ramifié présentant 1 à 6 atomes de carbone, un groupe aryle présentant 6 à 12 atomes de carbone ou aralkyle présentant 7 à 12 atomes de carbone et les groupes alkyles et aralkyles sont éventuellement substitués une à trois fois par des substituants halogènes.

**13.** Utilisation selon une ou plusieurs des revendications 9 à 12, **caractérisée en ce qu'**on utilise un composé aromatique, qui porte sur le noyau un substituant chloro ou bromo échangeable contre un atome de fluor, qui présente au moins groupe chloro ou bromo échangeable contre un atome de fluor en tant qu'autre substituant et éventuellement au moins un autre substituant pris dans le groupe comprenant F, $NO_2$, $CF_3$, CN, CHO, COF, COCl, $SO_2F$, $SO_2Cl$, $OCF_3$, $SCF_3$, $SO_2CF_3$, COOR, CONRR', $SO_2R$, COR, OR, -CO-O-CO- ou -CO-NR-CO-.

**14.** Utilisation selon une ou plusieurs des revendications 9 à 13, **caractérisée en ce qu'**on utilise un composé de formule générale (VI)

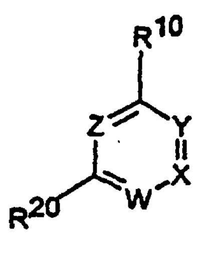

(VI)

où W représente un atome de N ou un groupe C-$R^{30}$, X représente un atome de N ou un groupe C-$R^{40}$, Y représente un atome de N ou un groupe C-$R^{50}$, Z représente un atome de N ou un groupe C-$R^{60}$, W, X et Y ne sont pas égaux à N en même temps, $R^{10}$, $R^{20}$, $R^{30}$, $R^{40}$, $R^{50}$, $R^{60}$ sont identiques ou différents et représentent des groupes H, F, Cl, Br, J, $NO_2$, NO, $CF_3$, CN, CHO, COF, COCl, $SO_2F$, $SO_2Cl$, $OCF_3$, $SCF_3$, $SO_2CF_3$, COOR, CONRR', $SO_2R$, COR, OR, un reste -CO-O-CO-, -CO-NR-CO- ou -CR"=CR"-CR"=CR"-, qui relie deux positions ortho, R et R' possèdent la signification donnée auparavant et R" sont indépendamment l'un de l'autre identiques ou différents et possèdent la même signification que $R^{10}$ à $R^{60}$, et au moins un des restes $R^{10}$ à $R^{60}$ représente un atome de chlore ou de brome.

**15.** Utilisation selon une ou plusieurs des revendications 9 à 14, **caractérisée en ce qu'**on utilise un composé de formule (VI), où seulement un des restes $R^{10}$ à $R^{60}$ représente un atome de chlore ou de brome, aucun des restes W, X, Y, Z ne représente un atome d'azote et au moins un des autres restes est pris dans le groupe comprenant $R^{10}$ à $R^{60}$ représentent des groupes $NO_2$, $CF_3$, CN, CHO, COF, COCl, $SO_2F$, $SO_2Cl$, $OCF_3$, $SCF_3$, $SO_2CF_3$, COOR, COONRR', $SO_2R$, COR, OR, -CO-O-CO-, -CO-NR-CO- ou -CR"=CR"-CR"=CR".

**16.** Utilisation selon une ou plusieurs des revendications 9 à 14, **caractérisée en ce qu'**on utilise un composé de formule (VI) où deux ou plusieurs des restes $R^{10}$ à $R^{60}$ représentent un atome de chlore ou de brome, les restes W, X, Y, Z représentent 0 à 3 atomes d'azote et les autres restes pris dans le groupe $R^{10}$ à $R^{60}$ peuvent tous représenter un atome d'hydrogène.

**17.** Utilisation selon une ou plusieurs des revendications 9 à 16, **caractérisée en ce qu'**on utilise en tant que fluorure de formule (V) le fluorure de calcium, le fluorure d'ammonium, le fluorure de lithium, le fluorure de sodium, le fluorure de potassium, le fluorure de rubidium, le fluorure de césium ou un mélange de ces composés.

**18.** Utilisation selon une ou plusieurs des revendications 9 à 17, **caractérisée en ce qu'**on utilise le fluorure de formule (V):équivalent de l'halogène échangeable dans un rapport de (0,5 à 10):(1), notamment de (0,8 à 5):(1), de préférence de (1 à 2):(1).